# EUROPEAN PATENT APPLICATION

(11) **EP 1 775 590 A1**
(43) Date of publication of application: **18.04.2007**
(21) Application number: 05384037.7
(22) Date of filing: 11.10.2005
(51) Int. Cl.: G01N 33/68, G01N 33/574

(54) **Non-invasive in vitro method to detect transitional cell carcinoma of the bladder**

(71) Applicant: LABORATORIOS S.A.L.V.A.T., S.A., 08950 Esplugues de Llobregat (Barcelona) (ES)
(72) Inventor: Soloaga Villoch, Ana, 48013 Bilbao (ES); Castrillo Diez, Jose Luis, 48993 Getxo (ES); Ramos Rodriguez, Inma, 48006 Bilbao (ES); Escuredo Garcia-Galdeano, Kepa, 48004 Bilbao (ES); Martinez Martinez, Antonio, 48100 Laukariz (ES); Simon Buela, Laureano, 20018 San Sebastián (ES)

(57) **Abstract**

The present invention refers to an non-invasive in vitro method to detect a transitional cell carcinoma of the bladder in an individual, to determine the stage or severity of this cancer in an individual or to monitor the effect of treatment administered to an individual suffering from this cancer.

## Description

### FIELD OF THE INVENTION

The present invention refers to a non-invasive in vitro method to detect the presence of a transitional cell carcinoma of the bladder in the urine of an individual, as well as to the use of peptide sequences derived from selected proteins and to a kit to perform the method.

### BACKGROUND OF THE INVENTION

Despite all the advances that have been achieved during the last 20 years, cancer is still one of the leading causes of mortality worldwide. Transitional cell carcinoma of the bladder (TCC) is the most common cancer of the urinary tract; it is also the fourth most common cancer in men and the eighth most common in women. According to the GLOBOCAN 2000 database of the International Agency for Research on Cancer, more than 136.000 new cases per year are diagnosed in Europe, 13.000 in Japan and 56.000 in North America. More than 3-4 times this number of patients are treated and monitored at hospitals every year; and more than 49.000, 4.500 and 12.000 deaths are due to bladder cancer every year in Europe, Japan and North America, respectively.

TCC is the most common type of bladder cancer, accounting for more than 90% of all cases. The remaining cases are squamous cell carcinomas (7%), adenocarcinomas (2%), and undifferentiated carcinomas (1%).

Tumor grade and stage are the best prognostic indicators of TCC. Bladder tumors are graded cytomorphologically from G1 to G3 in decreasing state of differentiation and increasing aggressiveness of the disease according to the World Health Organization (WHO). With respect to stage or invasivity, TCCs of the bladder are classified as superficial papillary (Ta and T1), muscle invasive (T2 to T4), or the uncommon carcinoma *in situ* or tumor *in situ* (TIS).

Low-grade (G1) tumors are usually confined to the mucosa or infiltrate superficial layers (stage Ta and T1). Most high-grade tumors are detected at least at T1 stage (invading lamina propria). Approximately 75% of the diagnosed bladder cancer cases are superficial. The remaining 25% are muscle invasive at the moment of diagnosis.

The clinical importance of distinguishing superficial and invasive tumors stems from the need to perform radical cystectomy, with lymphadenectomy and bladder reconstruction in case of extended cancers beyond the muscular layer. Tumors diagnosed in stages Ta and T1 allow the organ to be preserved and can be treated by transurethral resection and in some cases chemotherapy or intravesicular immunotherapy.

Patients with superficial TCC have a good prognosis but have a 70% risk of recurrence. These patients have to be monitored through invasive methods (i.e. cystoscopy) for tumor recurrence after treatment, following different time protocols depending on the hospital. The most frequent method is evaluation by the urologist every 3 months during the first 2 years, every 6 months for the following 2 years and every year thereafter. In spite of the high risk of recurrence, Ta tumors tend to be low grade and only 10-15% will progress to muscle invasion in 2 years. On the other hand, the percentage of T1 tumors that progresses to T2 stage is higher (30-50%).

Patients with invasive TCC have a poor prognosis; 50% of these patients at stage T2 or higher develop distant metastases within two years of diagnosis, and 69% of them die within 5 years. New diagnostic systems for early detection are needed given that 80-90 % of patients with T2 or higher are first diagnosed at this highly aggressive stage and not in previous stages (de Vere White, R.W. and Stapp, E., Oncology, 1998, 12:1717-1723).

Currently, the best diagnostic system for bladder cancer in individuals presenting symptoms such as hematuria or dysuria, in the absence of infection, is cystoscopy. Based on statistical data of incidence and recurrence, it has been estimated that more than 500.000 cystoscopies are performed annually in the USA (van Rhijn, B.W.G., et al., Cancer Res., 2001, 61:1265-1268). Flexible cystoscopes are used to make the technique less aggressive, but it remains invasive and highly unpleasant, and it also requires some form of anaesthesia.

The prevailing non-invasive technique for diagnosis of TCC is to identify neoplastic cells by morphological examination of the cells in urine (Loh, C.S., et al., Br. J. Urol., 1996, 77:655-658). Cytology is currently used to follow up patients diagnosed with and treated for bladder cancer. On the other hand urine cytology can detect tumors *in situ* that are not detectable by cystoscopy, as well as tumors located in the upper end of the bladder or the upper urinary tract, i.e. ureter, pelvis and kidney, that are not easily accessible by endoscopy (Lotan, Y. and Roehrborn, J. Urol., 2002, 167:75-79).

Nevertheless several studies have shown that cytology has a very low sensitivity for bladder cancer diagnosis, missing up to 50% of tumors (Boman, H., et al., J. Urol., 2002, 167:80-83). Findings from cytology studies are subtle and can be confused with reactive or degenerative processes. Cytology studies require expert, individual evaluation, which delays the availability of the results and further introduces subjectivity and variance to the final results. Actually, there is no highly sensible and specific non-invasive method available to diagnose bladder cancer (Boman, H., et al., J. Urol., 2002, 167:80-83).

Alteration of gene expression levels is tightly associated with uncontrolled cell growth and de-differentiation, common features of all cancers. The expression levels of the so-called "tumor suppressor genes", which act to block uncontrolled cell growth, are repressed in tumor cells; and expression levels of the so-called "oncogenes", which act to induce malignant growth, are elevated in tumor cells.

Many of these genes have been associated with bladder cancer development, including Rb, p53, p16, p14ARF and cyclin D1 (Fujimoto, K., et al., Cancer Res., 1998, 52:1393-1398; Grossman, B.H., et al., Clin. Cancer Res., 1998, 8:829-834; Balazs, M., et al., Genes Chromosomes Cancer, 1997, 19:84-89). The alteration in the expression of these genes may be used as a diagnostic marker of TCC; among these proposed markers have been proposed nuclear matrix protein NMP22 (Soloway, M.S., et al., J. Urol., 1996, 156:363-367; Casella, R., et al., J. Urol, 2000, 164:1926-1928), Hyaluronic Acid and Hyaluronidase (Pham, H.T., et al., Cancer Res., 1997, 57:778-783; Hautmann, S.H., et al., J. Urol., 2001, 165:2068-2074), Basement Membrane Complexes (BTA) (Pode, D., et al., J. Urol., 1999, 161:443-446; Thomas, L., et al., Clin. Chem, 1999, 45:472-477, Carcinoembryonic antigen (CEA) (Halim, A.B., et al., Int. J. Biol. Markers, 1992; 7:234-239), Uroplakin II (Wu, X.R., et al., Cancer Res., 1998; 58:1291-1297), Scatter Factor/Hepatocyte Growth Factor (SF/HGF) (Gohji, K., et al., J. Clin. Oncol., 2000; 18:2963-2971), proteins of the keratin/cytokeratin family like cytokeratin 20 (Buchumensky, V., et al., J. Urol., 1998, 160:1971-1974), cytokeratin 18 (Sánchez-Carbayo, M., et al., Clin. Cancer Res., 2000, 6:3585-3594), Mammary tumor 8-Ka Protein (MAT-8) (Morrison, B.W., et al., J. Biol. Chem., 1995, 270:2176-2182) and Telomerase (Lee, D.H., et al., Clinical Cancer Research, 1998, 4: 535-538).

However, it is likely that many of the genes involved in the initiation and progression of bladder cancer are currently unknown. No marker to predict the prognosis and extent of bladder cancer has been proven useful in clinical trials (Miyake, H., et al., J. Urol., 2002; 167:1282-1287).

### DESCRIPTION OF THE INVENTION

The current invention provides a highly sensitive, efficient and rapid non-invasive in vitro method concerning proteins associated with transitional cell carcinoma of the bladder. It further concerns a method for the diagnosis, prognosis and monitoring of transitional cell carcinoma of the bladder. A preferred embodiment of the invention concerns 38 differentially expressed biological markers that are of significant value for the detection, diagnosis, prognosis and/or monitoring of transitional cell carcinoma of the bladder.

Aminoacylase-1 (ACY1) is a cytosolic, homodimeric, zinc-binding enzyme that catalyzes the hydrolysis of acylated L-amino acids to L-amino acids and acyl group, and has been postulated to function in the catabolism and salvage of acylated amino acids. ACY1 has been assigned to chromosome 3p21.1, a region reduced to homozygosity in small-cell lung cancer (SCLC), and its expression has been reported to be reduced or undetectable in SCLC cell lines and tumors. The amino acid sequence of human aminoacylase-1 is highly homologous to the porcine counterpart, and ACY1 is the first member of a new family of zinc-binding enzymes.

Aldehyde reductase (AK1A1) belongs to the aldo/keto reductase family, it is involved in the reduction of biogenic and xenobiotic aldehydes and is present in virtually every tissue. It has been related to bladder cancer (Van Dijk B., et al., Eur. Urol. 2001, 40:509-14).

Alpha-amylase carcinoid (AMYC), pancreatic alpha-amylase (AMYP) and alpha amylase 1A (AMYS) belong to the glycosyl hydrolase 13 family and hydrolyze 1,4-alpha-glucoside bonds in oligosaccharides and polysaccharides, and thus catalyze the first step in digestion of dietary starch and glycogen. The human genome has a cluster of several amylase genes that are expressed at high levels in either salivary gland or pancreas.

Annexin IV (ANXA4) belongs to the annexin family of calcium-dependent phospholipid binding proteins. Although their functions are still not clearly defined, several members of the annexin family have been implicated in membrane-related events along exocytotic and endocytotic pathways. ANXA4 is almost exclusively expressed in epithelial cells.

Apolipoprotein A-I (APOA1) belongs to the apolipoprotein A1/A4/E family and participates in the reverse transport of cholesterol from tissues to the liver for excretion by promoting chloesterol efflux from tissues and by acting as a cofactor for the lecithin cholesterol acyltransferase (LCAT).

Biliverdin reductase A (BIEA) belongs to the gfo/idh/moca family and biliverdin reductase subfamily and reduces the gamma-methene bridge of the open tetrapyrrole, biliverdin ix alpha, to bilirubin with the concomitant oxidation of a NADH or NADPH.

Flavin reductase (BLVRB) catalyzes electron transfer from reduced pyridine nucleotides to flavins as well as to methylene blue, pyrroloquinoline quinone, riboflavin, or methemoglobin. BLVRB has a possible role in protecting cells from oxidative damage or in regulating iron metabolism. In the liver, it converts biliverdin to bilirubin. It is predominantly expressed in liver and erythrocytes, and at lower levels in heart, lung, adrenal gland and cerebrum.

Cathepsin D (CATD) is an acid protease which belongs to the peptidase A1 family and activates intracellular protein breakdown. It is involved in the pathogenesis of several diseases such as bladder cancer (Ozer E., et al., Urology 1999, 54:50-5 and loachim E., et al., Anticancer Res. 2002, 22:3383-8) breast cancer and possibly Alzheimer's disease.

Complement C3 precursor (CO3) plays a central role in the activation of the complement system. Its processing by C3 convertase is the central reaction in both complement pathways. CO3 has been related to urogenital tumors (Dunzendorfer U., et al., Eur. Urol. 1980, 6:232-6).

Cytosolic nonspecific dipeptidase (CPGL1) belongs to the peptidase M20A family and is also known as tissue carnosinase and peptidase A. It is a nonspecific dipeptidase rather than a selective carnosinase. It has not been related to cancer yet.

Alpha enolase (ENOA) is a multifunctional enzyme that, as well as its role in glycolysis, plays a part in various processes such as growth control, hypoxia tolerance and allergic responses. It may also function in the intravascular and pericellular fibrinolytic system due to its ability to serve as a receptor and activator of plasminogen on the cell surface of several cell-types such as leukocytes and neurones. Mammalian enolase is composed of 3 isozyme subunits, alpha, beta and gamma, which can form homodimers or heterodimers which are cell-type and development-specific. ENOA interacts with PLG in the neuronal plasma membrane and promotes its activation. It is used as a diagnostic marker for many tumors and, in the heterodimeric form, alpha/gamma, as a marker for hypoxic brain injury after cardiac arrest. It has been related to bladder cancer (Iczkowski K.A., et al., Histopathology 1999, 35:150-6).

Ferritin light chain (FRIL) belongs to the ferritin family and is the major intracellular iron storage protein in prokaryotes and eukaryotes. It is composed of 24 subunits of the heavy and light ferritin chains. Variation in ferritin subunit composition may affect the rates of iron uptake and release in different tissues. A major function of ferritin is the storage of iron in a soluble and nontoxic state.

Rab GDP dissociation inhibitor beta (GDIB) belongs to the rab gdi family and regulates the GDP/GTP exchange reaction of members of the rab family, small GTP-binding proteins of the ras superfamily, that are involved in vesicular trafficking of molecules between cellular organelles. GDIB is ubiquitously expressed. It has been related to bladder cancer (Seraj M.J., et al., Clin. Exp. Metastasis 2000, 18:519-25).

Plasma glutathione peroxidase (GPX3) belongs to the glutathione peroxidase family and catalyzes the reduction of hydrogen peroxide, organic hydroperoxide, and lipid peroxides by reduced glutathione and functions in the protection of cells against oxidative damage. Human plasma glutathione peroxidase has been shown to be a selenium-containing enzyme. GPX3 expression appears to be tissue-specific.

Gluthatione synthetase (GSHB) is important for a variety of biologic functions, including protection of cells from oxidative damage by free radicals, detoxification of xenobiotics and membrane transport.

Glutathione S-transferase (GSTP1) belongs to a family of enzymes that play an important role in detoxification by catalysing the conjugation of many hydrophobic and electrophilic compounds with reduced glutathione. It has been related to bladder cancer (Simic T., et al., Urology 2005, 65:1035-40 and Ryk C., et al., Int. J. Cancer 2005, 113:761-8).

Cytoplasmic isocitrate dehydrogenase (IDHC) belongs to the isocitrate and isopropylmalate dehydrogenase family and catalyzes the oxidative decarboxylation of isocitrate to form alpha-ketoglutarate.

Alterations in polymeric immunoglobulin receptor expression (among which Ig gamma-1 chain C region (IGHG1) is included) have been observed in bladder carcinoma (Rossel M., et al., Urol. Res. 1991, 19:361-6).

Vesicular integral-membrane protein VIP36 precursor (LMAN2) plays a role as an intracellular lectin in the early secretory pathway. Interacts with N-acetyl-D-galactosamine and high-mannose type glycans and may also bind to O-linked glycans. It is involved in the transport and sorting of glycoproteins carrying high mannose-type glycans.

Lymphocyte antigen 6 complex, locus 6 G6E (LY6G6E) belongs to the Lymphocyte antigen-6 superfamily. Members of this family are cystein-rich, generally GPI-anchored cell surface proteins, which have definite or putative immune related roles. Its specific function is unknown and has not been related to cancer yet.

Mannan-binding lectin serine protease 2, precursor (MASP2) belongs to the peptidase s1 family and is a trypsin protease that presumably plays an important role in the initiation of the mannose binding lectin (MBL) complement activation pathway. After activation it cleaves C4 generating C4A and C4B.

Nicotinate-nucleotide pyrophosphorylase (NADC) belongs to the nadC/mod D family and is an intermediate in the novo synthesis pathway of NAD from tryptophan and acts as a potent endogenous excitotoxin through hyperstimulation of N-methyl D-aspartate (NMDA) receptor in the neuron. Elevation of NADC levels in the brain has been linked to the pathogenesis of neurodegenerative disorders such as epilepsy, Alzheimer's disease and Huntington's disease. It has not been related to cancer yet.

Napsin A (NAPSA) belongs to the peptidase a1 family and it is expressed at the highest levels in the kidney, at a moderate level in the lung, and at low levels in the spleen and adipose tissue. It may be involved in processing of pneumocyte surfactant precursors.

Proliferation-associated protein 2G4 (PA2G4) is a single-stranded DNA-binding protein showing cell cycle specific variation in nuclear localization, which belongs to the peptidase m24c family. As the protein is expressed in response to mitogen stimulation, it may belong to a large family of cell cycle regulatory proteins or replication proteins that maintain the cell cycle activities of proliferating cells.

Oncogene DJ1 (PARK7) is highly expressed in pancreas, kidney, skeletal muscle, liver, testis and heart and acts as positive regulator of androgen receptor-dependent transcription. It may function as redox-sensitive chaperone and as sensor for oxidative stress. It prevents aggregation of SNCA and protects neurons against oxidative stress and cell death.

Poly(rc)-binding protein 1 (PCPB1) is a single stranded nucleic acid binding protein that binds preferentially to oligo dC. It may shuttle between the nucleus and the cytoplasm. It is abundantly expressed in skeletal muscle, thymus and peripheral blood leucocytes while a lower expression is observed in prostate, spleen, testis, ovary, small intestine, heart, liver, adrenal and thyroid glands.

Programmed cell death 6-interacting protein (PDC6I) is expressed in brain, heart, placenta, lung, kidney, pancreas, liver, skeletal muscle and cochlea and may play a role in the regulation of both apoptosis and cell proliferation. It interacts with PDCD6/ALG-2 and SH3KBP1.

Lysosomal acid phosphatase precursor (PPAL) belongs to the histidine acid phophatase family and hydrolyze orthophosphoric monoesters to alcohol and phosphate. It has been related to bladder cancer (Lin C.W., Cancer Res. 1991, 51:2710-9).

Peroxiredoxin 2 (PRDX2) belongs to the ahpc/tsa family and is involved in redox regulation of the cell. It reduces peroxides with reducing equivalents provided through the thioredoxin system. It is not able to receive electrons from glutaredoxin. It may play an important role in eliminating peroxides generated during metabolism. It might participate in the signalling cascade of growth factors and tumor necrosis factor-alpha by regulating the intracellular concentrations of H₂O₂. It enhances natural killer (NK) cells activity.

Glutaminyl-peptide cyclotransferase (QPCT) is responsible for the biosynthesis of pyroglutamyl peptides. It has a bias against acidic and tryptophan residues adjacent to a N-terminal glutaminyl residue and a lack of importance of chain length after the second residue.

Plasma retinol-binding protein (RETBP) belongs to the lipocalin family and is the specific carrier for retinol (vitamin A alcohol) in the blood. It delivers retinol from the liver stores to the peripheral tissues. In plasma, the RBP-retinol complex interacts with transthyretin which prevents its loss by filtration through the kidney glomeruli. A deficiency of vitamin A blocks secretion of the binding protein posttranslationally and results in defective delivery and supply to the epidermal cells. It has been related to bladder cancer (Basu T.K., et al., Eur. J. Cancer Clin. Oncol. 1982, 18:339-42).

Selenium-binding protein (SBP1) belongs to the selenium-binding protein family. Selenium is an essential nutrient that exhibits potent anticarcinogenic properties; a deficiency of selenium may cause certain neurologic diseases. It has been proposed that the effects of selenium in preventing cancer and neurologic diseases may be mediated by selenium-binding proteins.

Stress-induced-phosphoprotein 1 (STIP1) is an adaptor protein that mediates the association of the molecular chaperones HSC70 and HSP90 (HSPCA and HSPCB). It has not been related to cancer yet.

Transaldolase (TALDO) belongs to the transaldolase family and is a key enzyme of the nonoxidative pentose phosphate pathway providing ribose-5-phosphate for nucleic acid synthesis and NADPH for lipid biosynthesis. This pathway can also maintain glutathione at a reduced state and thus protect sulfhydryl groups and cellular integrity from oxygen radicals.

WD repeat protein 1 (WDR1) belongs to the wd-repeat aip1 family and induces dissassembly of actin filaments in conjunction with ADF/cofilin family proteins. It has not been related to cancer yet.

One aspect of the present invention relates to a non-invasive in vitro method that comprises:
a) the detection and/or quantification of one or more proteins selected from ACY1, AK1A1, AMYC, AMYP, AMYS, ANXA4, APOA1, BIEA, BLVRB, CATD, C03, CPGL1, ENOA, FRIL, GDIB, GPX3, GSHB, GSTP1, IDHC, IGHG1, LMAN2, LY6G6E, MASP2, NADC, NAPSA, PA2G4, PARK7, PCBP1, PDC61, PPAL, PRDX2, PTD012, QPCT, RETBP, SBP1, STIP1, TALDO and WDR1 in a urine sample from an individual; and/or
b) the quantification of one or more ratios between the amount of one or more proteins selected from ACY1, AK1A1, AMYC, AMYP, AMYS, ANXA4, APOA1, BIEA, BLVRB, CATD, CO3, CPGL1, ENOA, FRIL, GDIB, GPX3, GSHB, GSTP1, IDHC, IGHG1, LMAN2, LY6G6E, MASP2, NADC, NAPSA, PA2G4, PARK7, PCBP1, PDC6l, PPAL, PRDX2, PTD012, QPCT, RETBP, SBP1, STIP1, TALDO and WDR1 in a urine sample from an individual, and one or more proteins independently selected from the group comprising constantly expressed proteins and differentially expressed proteins in the same urine sample. Differential regulation of these proteins is an indicator of transitional cell carcinoma of the bladder. Further, urine samples are very diverse and the protein concentration may vary depending on several factors. The ratios between proteins allow for overcoming this variance.

Another aspect of the invention relates to the use of one or more peptide sequences derived from one or more proteins selected from ACY1, AK1A1, AMYC, AMYP, AMYS, ANXA4, APOA1, BIEA, BLVRB, CATD, CO3, CPGL1, ENOA, FRIL, GDIB, GPX3, GSHB, GSTP1, IDHC, IGHG1, LMAN2, LY6G6E, MASP2, NADC, NAPSA, PA2G4, PARK7, PCBP1, PDC6l, PPAL, PRDX2, PTD012, QPCT, RETBP, SBP1, STIP1, TALDO and WDR1 to detect the presence of transitional cell carcinoma of the bladder, to determine the stage or severity of this cancer, to assess the lack of disease after surgical resection, to establish the diagnosis and/or prognosis of this cancer and/or to monitor the effect of the treatment administered to an individual suffering from said cancer.

Another aspect of the invention relates to the use of one or more nucleotides or peptide sequences derived from one or more proteins selected from ACY1, AK1A1, AMYC, AMYP, AMYS, ANXA4, APOA1, BIEA, BLVRB, CATD, CO3, CPGL1, ENOA, FRIL, GDIB, GPX3, GSHB, GSTP1, IDHC, IGHG1, LMAN2, LY6G6E, MASP2, NADC, NAPSA, PA2G4, PARK7, PCBP1, PDC6I, PPAL, PRDX2, PTD012, QPCT, RETBP, SBP1, STIP1, TALDO and WDR1, alone or in any combination, in methods to screen for, identify, develop and evaluate the efficiency of compounds to transitional cell carcinoma of the bladder.

Another aspect of the invention relates to a kit to perform the method as previously defined comprising any combination of antibodies that specifically recognises one or more of these proteins and a carrier in suitable packaging.

For the purposes of the present invention the following definitions have been used:

The term "38 proteins of the invention" refers to the group of proteins comprising ACY1, AK1A1, AMYC, AMYP, AMYS, ANXA4, APOA1, BIEA, BLVRB, CATD, CO3, CPGL1, ENOA, FRIL, GDIB, GPX3, GSHB, GSTP1, IDHC, IGHG1, LMAN2, LY6G6E, MASP2, NADC, NAPSA, PA2G4, PARK7, PCBP1, PDC6l, PPAL, PRDX2, PTD012, QPCT, RETBP, SBP1, STIP1, TALDO and WDR1.

The term "individual" refers to all species of animals classified as mammals and includes, but is not restricted to, domestic and farm animals, primates and humans, and preferably refers to a male or female human of any age or race.

The term "cancer" refers to the disease that is typically characterised by abnormal or unregulated cell growth, capable of invading adjacent tissues and spreading to distant organs.

The term "carcinoma" refers to the tissue resulting from abnormal or unregulated cell growth.

The term "transitional cell carcinoma of the bladder" or its abbreviation "TCC" refer to any malign proliferative disorder in bladder epithelial cells.

The term "tumor" refers to any abnormal mass of tissue generated by a neoplastic process, whether this is benign (non cancerous) or malignant (cancerous).

The term "differentially expressed proteins" refers to those proteins which are expressed differently in the urine from a healthy individual and in the urine from a patient of transitional cell carcinoma. In the present invention the group of proteins comprised by differentially expressed proteins includes both the 38 proteins of the invention, as well as any protein of a urine sample, whose ratio changes less than two-fold when comparing two different urine samples: one from a healthy individual and one from a patient of transitional cell carcinoma, this quantification being carried out by the Phoretix^{™} software. The term "differentially expressed protein" may refer to a protein, whose expression is either induced or repressed.

The term "constantly expressed proteins" refers to those proteins, whose expression level is the same in the urine form a healthy individual and in the urine from a patient of transitional cell carcinoma. In the literature there are many examples of these proteins, whose content in urine is always constant.

The term "inversely expressed" refers to two differentially expressed proteins whose expression patterns are opposite (that is, in a sample one is overexpressed while the other is repressed or vice versa).

The term "healthy individual" refers an individual who is not currently suffering from transitional cell carcinoma. The term healthy individual may include patients from other urologic diseases.

The term "previously diagnosed" relates to an individual who has already received a first positive diagnostic for and has been treated against TCC, including healthy individuals, as well as recurrantpatients.

The term "not previously diagnosed" relates to an individual who has never received a first positive diagnostic for TCC *(de novo* diagnostic).

The term "treatment" refers to any process, action, application or the like, wherein an individual is subject to medical aid with the object of improving his condition, directly or indirectly.

The term "specificity" refers to the ability of a test to exclude the presence of a disease when it is truly not present. Specificity is the proportion of non-diseased patients for whom there is a correctly negative test, expressed as the number of true negatives divided by the sum of true negatives and false positives.

The term "sensitivity" refers to the ability of a test to detect a disease when it is truly present. Sensitivity is the proportion of all diseased patients for whom there is a positive test, determined as the number of the true positives divided by the sum of true positives and false negatives.

The term "gene" refers to a region of a molecular chain of deoxyribonucleotides that encodes a protein and may represent a portion of a coding sequence or a complete coding sequence.

The term "protein" refers to at least one molecular chain of amino acids linked intermolecularly through either covalent or non-covalent bonds. The term includes all forms of post-translational protein modifications, for example glycosylation, phosphorylation or acetylation.

The terms "peptide" and "polypeptide" refer to molecular chains of amino acids that represent a protein fragment. The terms "protein" and "peptide" are used indistinguishably.

The term "antibody" refers to a Y-shaped protein (known as immunoglobulin) on the surface of B cells that is secreted into the blood or lymph in response to an antigenic stimulus , such as an exogenous protein, bacterium, virus, parasite, or transplanted organ, and that exhibits a specific binding activity for a target molecule called an "antigen". The antigen binding region of immunoglobulins can be divided into either F(ab')₂ or Fab fragments. The term "antibody" includes monoclonal and polyclonal antibodies, either intact or fragments derived from them; and includes human antibodies, humanised antibodies and antibodies of non-human origin. The "monoclonal antibodies" are homogeneous, highly specific antibody populations directed against a single antigenic site or "determinant" of the target molecule. "Polyclonal antibodies" include heterogeneous antibody populations that are directed against different antigenic determinants of the target molecule.

The term "specific antibody" refers to an antibody generated against a specific protein (in this case against a particular bladder cancer marker).

The term "complex formed by the antibodies" refers to a complex formed by an antigen and its specific antibody.

The term "combibody" (combinatorial antibody) refers to an antibody displayed on filamentous phages, which allows direct screening of cDNA libraries for expression of cell-surface-reactive antibodies, without the need for antibody production and purification using bacteria or eukaryotic cell systems.

The term "Fab recombinant antibody" refers to a recombinant antibody that only contains the Fab fragment that is univalent and useful when the antibody has a very high avidity for its antigen. They can be recombinantly obtained if the protein sequence is known.

The term "ScFv antibody fragment" refers to a single chain variable fragment (scFv) that can be expressed in bacterial cultures.

The term "epitope" refers to an antigenic determinant of a protein, which is the sequence of amino acids of the protein that a specific antibody recognises. Such epitopes may be comprised of a contiguous stretch of amino acids (linear epitope) or of non-contiguous amino acids that are brought into proximity with one another by virtue of the three dimensional folding of the polypeptide chain (discontinuous epitopes).

The term "solid phase" refers to a non-aqueous matrix to which the antibody can bind. Examples of materials for the solid phase include but are not limited to glass, polysaccharides (for example agarose), polyacrylamide, polystyrene, polyvinylic alcohol and silicons. Examples of solid phase forms are the well of a plate or a purification column.

The term "dipstick" refers to a device dipped into a liquid to perform some kind of test that may determine and/or quantify some properties of the liquid (chemical, physical, etc). This kind of dipstick is usually made of paper or cardboard and is impregnated with reagents whose colour changes indicate some feature of the liquid.

The term "carrier" refers to a mechanism or device by which something is conveyed or conducted.

The term "packaging" refers to the containment and packing prior to sale with the primary purpose of facilitating the purchase and use of a product.

One embodiment of the invention relates to a non-invasive in vitro method that comprises:
a) the detection and/or quantification of one or more proteins selected from the 38 proteins of the invention in a urine sample from an individual; and/or
b) the quantification of one or more ratios between the amount of one or more proteins selected from the 38 proteins of the invention in a urine sample from an individual, and one or more proteins independently selected from the group comprising constantly expressed proteins and differentially expressed proteins in the same urine sample; this method being employed to detect the presence of transitional cell carcinoma of the bladder, to determine the stage or severity of this cancer, to assess the lack of disease after surgical resection, to establish the diagnosis and/or prognosis of this cancer and/or to monitor the effect of the treatment administered to an individual suffering from said cancer.

Another embodiment relates to a non-invasive in vitro method that comprises the quantification of one or more ratios between the amount of one or more proteins selected from the 38 proteins of the invention in a urine sample from an individual, and one or more proteins independently selected from the group comprising constantly expressed proteins and differentially expressed proteins in the same urine sample.

Another embodiment relates to a non-invasive in vitro method that comprises the quantification of one or more ratios between two different proteins selected from the 38 proteins of the invention in a urine sample from an individual.

Another embodiment relates to a non-invasive in vitro method that comprises the quantification of one or more ratios between two different proteins selected from the 38 proteins of the invention in a urine sample from an individual, wherein the two different proteins are inversely expressed.

In another embodiment the stage of development of the bladder cancer is determined by quantitative determination of one or more proteins selected from the 38 proteins of the invention either alone or in combination in the same sample.

In another embodiment the method allows to determine the progression of the disease when the same protein is compared from different samples obtained at different times.

In another embodiment one or more proteins selected from the 38 proteins of the invention may be used to monitor the efficacy of pharmacological or surgical treatment.

In another embodiment the sample to be analyzed is obtained from an individual not previously diagnosed with transitional cell carcinoma of the bladder.

In another embodiment the sample to be analyzed is obtained from an individual who has been previously diagnosed with transitional cell carcinoma of the bladder.

In another embodiment the sample to be analyzed is obtained from an individual currently receiving treatment against transitional cell carcinoma of the bladder.

In another embodiment the method comprises obtaining an extract of proteins of the sample.

In another embodiment the detection and/or quantification of proteins comprises a first step, in which the protein extract of the sample is placed in contact with a composition of one or more specific antibodies against one or more epitopes of the protein or proteins, and a second step, in which the complexes formed by the antibodies and the proteins are quantified.

In another embodiment the specific antibodies used for the detection of proteins are of human origin, humanised or of non-human origin and selected from monoclonal or polyclonal antibodies, intact or recombinant fragments of antibodies, combibodies and Fab or scFv antibody fragments.

In another embodiment the techniques used for the detection and/or quantification of the complexes formed by antibodies and proteins comprise: western-blot, ELISA (Enzyme-Linked Immunosorbent assay), RIA (Radioimmunoassay), Competitive EIA (Competitive Enzyme Immunoassay), DAS-ELISA (Double Antibody Sandwich-ELISA), immunocytochemical or immunohistochemical techniques, techniques based on the use of biochips or protein microarrays that include specific antibodies, assays based on the precipitation of colloidal gold in formats such as dipsticks; or by affinity chromatography techniques, ligand binding assays or lectin binding assays.

Another embodiment relates to the use of one or more sequences derived from one or more proteins selected from the 38 proteins of the invention to detect the presence of transitional cell carcinoma of the bladder, to determine the stage or severity of this cancer, to assess the lack of disease after surgical resection, to establish the diagnosis and/or prognosis of this cancer and/or to monitor the effect of the treatment administered to an individual suffering from said cancer refers, wherein the proteins are present in urine.

Another embodiment relates to the use of one or more nucleotides or peptide sequences derived from one or more proteins selected from the 38 proteins of the invention, alone or in any combination, in methods to screen for, identify, develop and evaluate the efficiency of compounds to transitional cell carcinoma of the bladder, wherein the proteins are present in urine.

Another embodiment relates to a kit to perform a method as previously defined comprising any combination of antibodies that specifically recognises one or more of these proteins and a carrier in suitable packaging, the kit being employed to detect the presence of transitional cell carcinoma of the bladder, to determine the stage or severity of this cancer, to assess the lack of disease after surgical resection, to establish the diagnosis and/or prognosis of this cancer and/or to monitor the effect of the treatment administered to an individual suffering from said cancer.

Another embodiment relates to a kit to perform a method as previously defined comprising a biochip.

Another embodiment relates to a kit to perform a method as previously defined comprising a biochip, wherein the biochip comprises antibodies for the detection of proteins selected from ACY1, AK1A1, AMYC, AMYP, AMYS, ANXA4, APOA1, BIEA, BLVRB, CATD, CO3 CPGL1, ENOA, FRIL, GDIB, GPX3, GSHB, GSTP1, IDHC, IGHG1, LMAN2, LY6G6E, MASP2, NADC, NAPSA, PA2G4, PARK7, PCBP1, PDC61, PPAL, PRDX2, PTD012, QPCT, RETBP, SBP1, STIP1, TALDO and WDR1, constantly expressed proteins and differentially expressed proteins.

There is a wide range of immunological assays available to detect and/or quantify the formation of specific antigen-antibody complexes; numerous competitive or non-competitive protein-binding assays have been described previously and a large number of these are commercially available. Hence, proteins can be quantified with antibodies such as, for example: monoclonal antibodies, polyclonal antibodies, either intact or recombinant fragments of these, combibodies and Fab or scFv fragments of antibodies, specific for proteins. These antibodies may be of human origin, humanised or of animal origin. On the other hand, they can be labelled or unlabelled and can be used in a wide range of assays. Marker molecules that can be used to label antibodies include radionuclides, enzymes, fluorophores, chemoluminescent reagents, enzymatic substrates or cofactors, enzymatic inhibitors, particles, colorants and derivatives. The higher the antibody binding specificity, the lower the antigen concentration that can be detected.

There are a wide variety of assays well known to those skilled in the art that can be used in the present invention, which use unlabelled antibodies (primary antibody) and labelled antibodies (secondary antibodies); these techniques include but are not limited to the western-blot or western transfer, ELISA (Enzyme-Linked immunosorbent assay), RIA (Radioimmunoassay), Competitive EIA (Competitive enzyme immunoassay), DAS-ELISA (Double antibody sandwich-ELISA), immunocytochemical and immunohistochemical techniques, techniques based on the use of biochips or protein microarrays that include specific antibodies or colloidal precipitation in formats such as dipsticks. Other ways to detect and/or quantify proteins include affinity chromatography techniques, ligand binding assays or lectin binding assays. The preferred embodiments of this aspect of the invention are protein microarrays and double antibody sandwich ELISA (DAS-ELISA). In these immunoassays any antibody, or combination of antibodies can be used, that are specific against one or more epitopes of the 38 proteins of the invention. As an example of one of the many possible formats of this assay, a monoclonal or polyclonal antibody, or a fragment of this antibody, or a combination of these antibodies that recognise one or more epitopes of the 38 proteins of the invention are attached to the surface of a solid phase support and placed in contact with the sample to be analyzed and incubated for a specific time and in appropriate conditions to form the antigen-antibody complexes. After washing in appropriate conditions to eliminate non-specific complexes, an indicator reagent, consisting in a monoclonal or polyclonal antibody, or a fragment of this antibody, or a combination of these and which recognises one or more epitopes of the 38 proteins of the invention, bound to a signal generating molecule, is incubated with the antigen-antibody complexes in appropriate conditions of time and temperature. The presence of one or more proteins selected from the 38 proteins of the invention in the sample to be analyzed is detected and, if present, quantified and the signal generated is measured. The amount of protein present in the sample to be analyzed is proportional to these signals, and thus correcting for variance of protein concentrations between samples.

As indicated above, the method of the invention involves monitoring the stage of bladder carcinoma by quantitating the differentially-expressed soluble proteins within a urine sample through specific antibodies. As will be appreciated by those skilled in the art, any means for specifically identifying and quantifying these proteins are contemplated.

In the description which follows, the generalized method for obtaining and analyzing the total protein content of human urine samples (herein referred to as the sample) is disclosed. The method involves the manipulation and preparation of the samples, the use of 2D electrophoresis to separate proteins within the sample, the selection of differentially expressed proteins by means of image analysis and statistics of different samples and the use of one or more of the differentially expressed proteins of the invention to generate protein-specific antibodies to be used as bladder cancer markers.

The comparative proteome analysis was performed between samples obtained from healthy individuals (controls) and patients diagnosed with TCC (Ta, T1-low grade, T1-high grade and T2) in an attempt to identify differentially expressed proteins in the various stages of bladder cancer and during bladder cancer progression. Among all the proteins that showed differential expression, 38 proteins changed more than two-fold reproducibly. They were identified by peptide mass fingerprinting using mass spectrometry and database search.

### I.Identification of differentially expressed proteins

To identify differentially expressed proteins along progression of bladder cancer, protein profiles of healthy urines were compared with those of early-stage and advanced bladder tumors patients using a proteomic approach.

Urine samples (150 in total) were collected from healthy individuals and from patients with transitional bladder cancer visiting the Urology units of Hospitals belonging to the Spanish Public Health Network. These samples were classified as follows:
a) No Carcinoma (63 samples) including:
   - Controls (CV, 32 samples): patients that had had bladder cancer and had been resected from the tumor and were being controlled (cystoscopy showed no other affectations).
   - Patients with other urological tract diseases (31 samples, including among others prostate benign hyperplasia, prostate cancer).
b) Transitional cell carcinoma patients (87 samples): patients diagnosed with the disease at different stages of development including:
   - Ta (21 samples)
   - T1-low grade (29 samples)
   - T1-high grade (19 samples)
   - T2 (18 samples)

The TCC group of samples was accompanied by a biopsy that was the key for their classification in the stages of development of bladder cancer.

### A. Manipulation of urine samples and separation of proteins.

Urine samples were frozen at -80°C and shipped to the lab in dry ice without breaking the cold chain. Samples were kept at -80°C until they were processed. The samples were very heterogenic, ranging from light yellow urines to red ones with blood crumps, transparent or containing tissues in suspension. The total volume was also very variable, from 5 to 100 mL. The protein concentration of the samples ranged from 20 µg/mL to 2 mg/mL (150 µg/mL was the average concentration). To obtain the protein content, samples were thawed on ice and centrifuged at 2000xg for 5 min at 4°C. The supernatant was used to determine protein concentration. The volume necessary to precipitate 100 µg of protein was calculated taking into account that the efficacy of protein precipitation with 15% w/v of trichloroacetic acid (TCA) is 75%. The rest of the urine sample was frozen again at -80°C and stored for further 2D electrophoresis (if needed). TCA and urine were mixed for 1 hour on ice, and then centrifuged at 16000xg for 20 min at 4°C to obtain the pellet of precipitated proteins. This pellet was washed with acetone stored at -20°C and dried by solvent evaporation.

To perform the two dimension (2D) electrophoresis experiments, the first dimension was IEF (isoelectric focusing), where proteins separate by their charge (pl); the second dimension was SDS-PAGE where proteins separate by their molecular weight. To carry out the first dimension, the dried pellet of proteins was resuspended with 450 µl of rehydratation buffer (Urea 7M, Thiourea 2M, CHAPS 2%, IPG buffer 2%, bromophenol blue 0.002%) for 1 hour at room temp. IPG buffer (Amersham, ref# 17-600-88) was used so that the IEF ranged from pH 3-10. For the IEF, the Ettan^{™} IPGphor^{™} Isoelectric Focusing System from Amersham was used following the manufacturer's directions. The IEF was performed in immobilized pH gradients, named IPG strips, purchased from Amersham (ref# 17-6002-45). The solubilized proteins focused in the first dimension in the strips after 16 hours of active rehydratation of the gel at 30 V. Then the voltage was increased to 8000 V, the intensity never being higher than 50 µA per gel. The IEF was finished when the voltage reached 90000 Vhour.

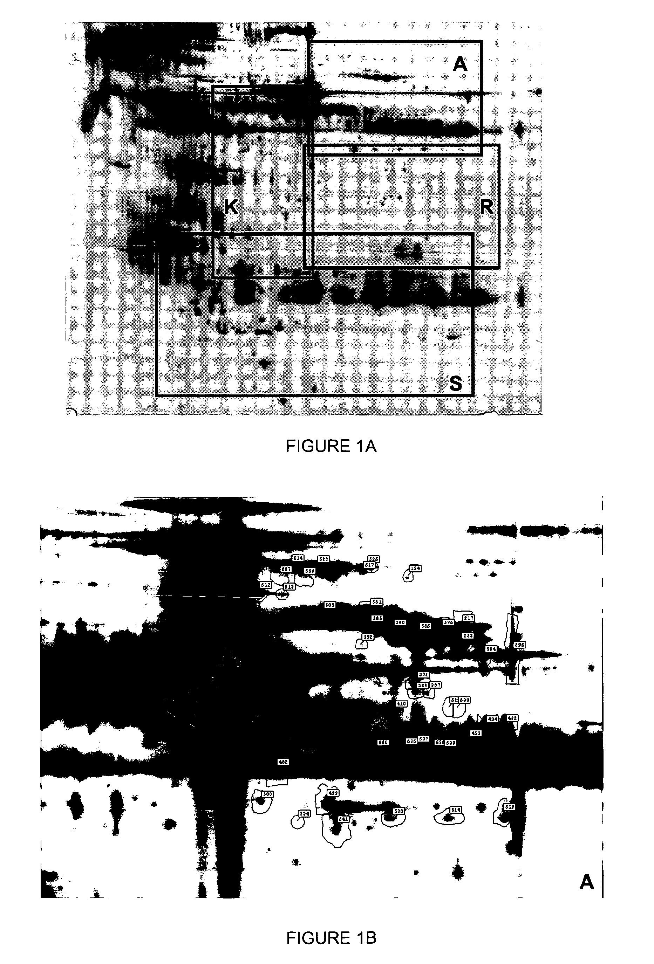

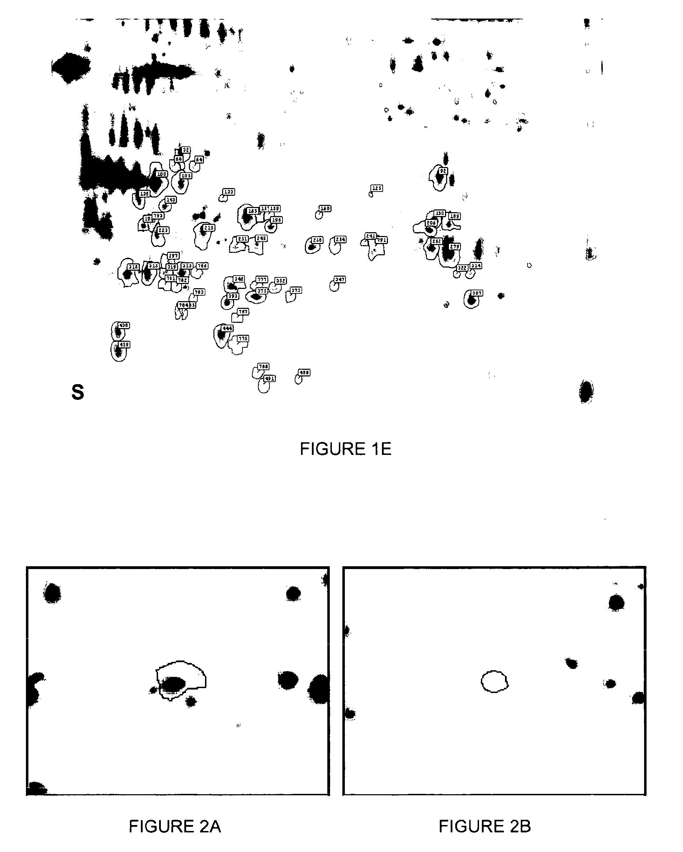

For the second dimension, 26 x 20 cm 12.5% acrylamide were polymerized in the lab using the Ettan DALT twelve Gel Caster from Amersham. Gels were run in the Ettan DALT twelve Large Format Vertical System and following the manufacturer's instructions until the electrophoresis front left the gel. Gels were silver nitrate dyed using the dye kit from Amersham (17-1150-01) following the manufacturer's directions. Gels were then dried and stored for subsequent image analysis of the protein spots (FIGURE 1A).

For some urine samples more than one 2D-gel was run.

### B. Analyses of the protein spots on the gels

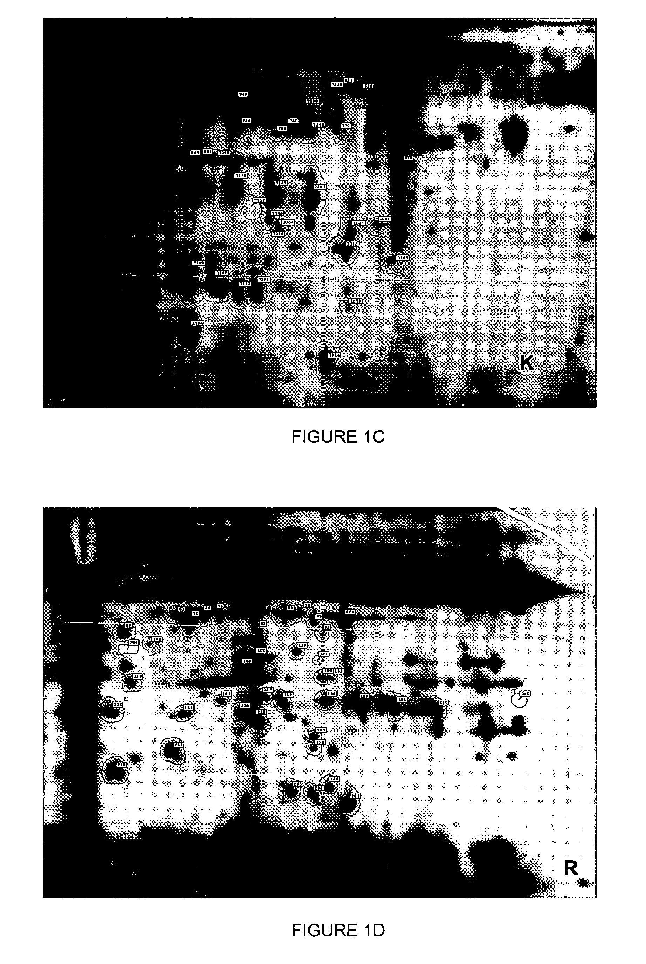

Every gel was scanned to obtain the map of spots for image analysis. Phoretix^{™} 2D Evolution software, version 2004, from Nonlinear Dynamics (UK) was used to analyze image files in a 300 dpi (dot per inch) format and 8 bits/channel. To increase its resolution, the analysis was performed in discrete areas of the gels. From each area, named as A (FIGURE 1 B), K (FIGURE 1 C), R (FIGURE 1 D) and S (FIGURE 1 E), the best gels were selected so that the samples belonging to the different groups be represented as long as the statistics would be performed with the values obtained from the selected gels. The Phoretix^{™} 2D Evolution software transformed the information of the flat image into a 3D image, where the intensity of each spot correlated with its volume and with the relative amount of the correspondent protein in the urine of the individual. With the software tables of intensities for each spot were obtained in each gel. These raw data were the basis for the subsequent statistical analyses.

To perform the statistical analysis of each individual spot and compare its intensity in two different groups (for instance, CV and Ta), a normal distribution had to be proven in these blocks of data. To compare the spots belonging to two groups, a Student's test was applied and a p value obtained: this was the theoretical error of assigning a spot to one subgroup or to another one. Only the spots with p values ≤0.05 were selected. The fold change or average ratio of these spots was calculated either referred to the total spot volume of the sample, that is the theoretical total protein content; or to the a constantly expressed protein, the amount of which is constant in every sample; or to a second differentially expressed protein of the same sample.

Also the n value or number of samples was taken into account.

38 proteins that showed statistical significance and robustness in their fold change when comparing urines from control patients with urines from patients diagnosed with transitional bladder cancer.
The identification of those spots was carried out by MALDI-ToF (Matrix-Assisted Laser-Desorption/lonization Time Of Flight) spectroscopy. Those urine samples whose 2D electrophoresis had shown statistically significant spots were submitted again to 2D electrophoresis and the spots excised from the gel. Proteins were digested and analyzed by MALDI-TOF spectrometer. Peptide mass fingerprint enabled the identification of 38 proteins, that were identified as: ACY1, AK1A1, AMYC, AMYP, AMYS, ANXA4, APOA1, BIEA, BLVRB, CATD, CO3 CPGL1, ENOA, FRIL, GDIB, GPX3, GSHB, GSTP1, IDHC, IGHG1, LMAN2, LY6G6E, MASP2, NADC, NAPSA, PA2G4, PARK7, PCBP1, PDC61, PPAL, PRDX2, PTD012, QPCT, RETBP, SBP1, STIP1, TALDO and WDR1 (see table 1). Thus, these 38 proteins, that had proven to be differentially expressed in patients diagnosed with TCC, were identified as biological markers of significant value for the diagnosis, prognosis and treatment monitoring of the disease.

**Table 1**

| **PROTEIN SYMBOL** | **SEQ ID No** | **SPOT #** | **GI** | **UniProt** | **OMIM** | **PROTEIN NAME (S):** |
|---|---|---|---|---|---|---|
| **ACY1** | 1 | K878 | 4501901 | Q03154 | 104620 | Aminoacylase-1 |
| **AK1A1** | 2 | R183 | 1633300 | P1450 | 103830 | Aldehyde reductase |
| **AMYC** | 3 | A638 | 10280622 | P19961 | 104660 | Alpha-amylase carcinoid |
| **AMYP** | 4 | A636 | 4502085 | P04746 | 104650 | Pancreatic alpha-amylase |
| **AMYS** | 5 | A637 | 1633119 | P04745 | 104700 | Alpha-amylase 1A (salivary |
| **ANXA4** | 6 | K1273 | 1703319 | P09525 | 106491 | Annexin A4 |
| **APOA1** | 7 | S313 | 178775 | P02647 | 107680 | Apolipoprotein A-1 |
| | | S319 | | | | |
| | | S782 | | | | |
| **BIEA** | 8 | R211 | 47117734 | P53004 | 109750 | Biliverdin reductase A |
| **BLVRB** | 9 | S314 | 32891807 | P30043 | 600941 | Flavin reductase |
| | | S322 | | | | |
| **CATD** | 10 | K754 | 30584113 | P07339 | 116840 | Cathepsin D |
| | | K760 | | | | |
| **C03** | 11 | A594 | 4557385 | P01024 | 120700 | Complement C3 (fragment) |
| | 12 | A596 | 40786791 | | | |
| **CPGL1** | 13 | K629 | 15620780 | Q96KP4 | 169800 | Cytosolic nonspecific dipeptidase |
| **ENOA** | 14 | R383,A519 | 4503571 | P06733 | 172430 | Alpha enolase |
| | 15 | R058, A524 | 31873302 | | | |
| | 16 | R054, A530 | 30583767 | | | |
| **FRIL** | 17 | S444 | 182516 | P02792 | 134790 | Ferritin light chain |
| **GDIB** | 18 | A499, R061 | 6598323 | P50395 | 600767 | Rab GDP dissociation inhibitor beta |
| **GPX3** | 19 | S777 | 404108 | P22352 | 138321 | Plasma glutathione peroxidase |
| **GSHB** | 20 | K7230 | 8248826 | P48637 | 601002 | Glutathione synthetase |
| **GSTP1** | 21 | S346 | 20664359 | P09211 | 134660 | Glutathione S-transferase P |
| **IDHC** | 22 | R119 | 49168486 | O75874 | 147700 | Cytoplasmic Isocitrate dehydrogenase |
| **IGHG1** | 23 | R195 | 46326410 | P01857 | 147100 | Ig gamma-1 chain C region |
| **LMAN2** | 24 | K1187 | 5803023 | Q12907 | No | Vesicular integral-membrane protein VIP36 precursor |
| | | K7280 | | | | |
| | | K7292 | | | | |
| **LY6G6E** | 25 | K7359 | 55961604 | Q5SRS9 | No | Lymphocyte antigen 6 complex |
| **MASP2** | 26 | S775 | 50513646 | 000187 | 605102 | Mannan-binding lectin serine protease 2 |
| **NADC** | 27 | K1166 | 14603130 | Q15274 | 606248 | Nicotinate-nucleotide pyrophosphorylase |
| **NAPSA** | 28 | K1213 | 17389633 | 096009 | 605631 | Napsin A |
| | | R276 | | | | |
| **PA2G4** | 29 | R056 | 33879698 | Q9UQ80 | 602145 | Proliferation-associated protein 2G4 |
| **PARK7** | 30 | S347 | 50513593 | Q99497 | 602533 | Oncogene DJ1; DJ-1 |
| **PCBP1** | 31 | R149 | 5453854 | Q15365 | 601209 | Poly(rC)-binding protein 1 |
| **PDC6I** | 32 | A627 | 46249756 | Q8WUM4 | 608074 | Programmed cell death 6-interacting protein |
| **PPAL** | 33 | A641, R072 | 32700070 | P11117 | 171650 | Lysosomal acid phosphatase precursor |
| **PRDX2** | 34 | S393 | 1617118 | P32119 | 600538 | Peroxiredoxin 2 |
| **PTD012** | 35 | R216 | 48257065 | AAD40375 | None | PTD012 protein |
| **QPCT** | 36 | K7258 | 525241 | Q16769 | 607065 | Glutaminyl-peptide cyclotransferase |
| | | K7346 | | | | |
| | | K7347 | | | | |
| **RETBP** | 37 | S784 | 230284 | P02753 | 180250 | Plasma retinol-binding protein |
| | | S785 | | | | |
| **SBP1** | 38 | A482 | 1374792 | Q13228 | 604188 | Selenium-binding protein 1 |
| **STIP1** | 39 | A387 | 54696884 | P31948 | 605063 | Stress-induced-phosphoprotein 1 |
| **TALDO** | 40 | R205 | 16307182 | P37837 | 602063 | Transaldolase |
| | | R206 | | | | |
| **WDR1** | 41 | A372 | 3420181 | 075083 | 604734 | WD-repeat protein 1 |

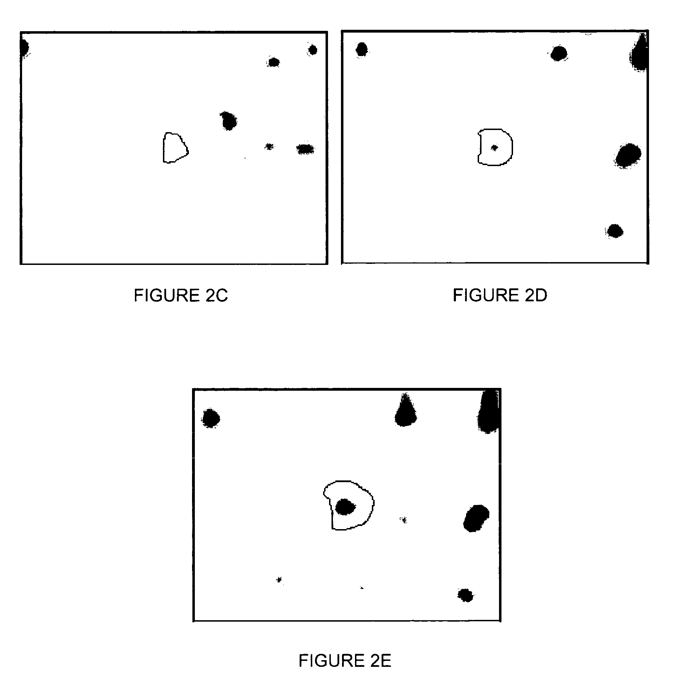

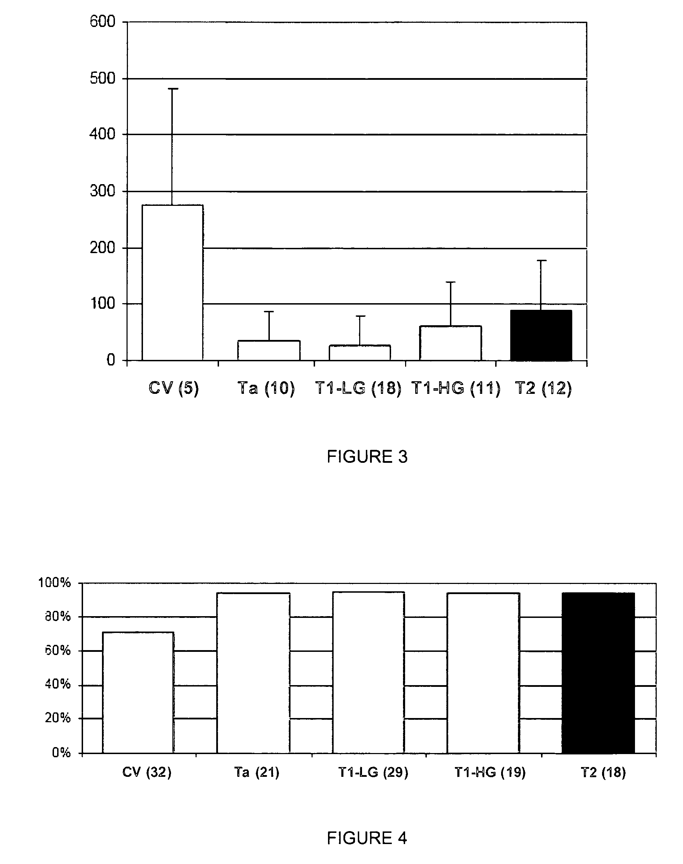

As an example, FIGURE 2 shows spot R211 corresponding to BIEA in the R area of bidimensional electrophoresis (2D) gel obtained from urine samples of a healthy individual (FIGURE 2A), of a patient suffering cancer at the Ta stage (FIGURE 2B), of a patient suffering cancer at the T1-low grade stage (FIGURE 2C), of a patient suffering cancer at the T1-high grade stage (FIGURE 2D) and of a patient suffering cancer at the T2 stage (FIGURE 2E). It can be seen that the intensity of spot R211 is clearly decreased in the samples of the patients with cancer at different stages, when compared to the healthy urine sample. These differences showed to be significant after statistical analysis with Phoretix^{™}. FIGURE 3 shows the intensity of spot R211 in samples CV, Ta, T1-low grade, T1-high grade and T2. The number of samples for each group is indicated in parentheses. FIGURE 4 shows the robustness of the intensity measurement for spot R211 in different urine samples. This is expressed as the percentage of the gels that maintain the presence or absence of spot R211 in every gel analyzed. The number of samples for each group is again indicated in parentheses. Table 2 shows the p and fold change values of the statistical analysis for spot R211 in samples of different stages of cancer in comparison to a sample of a non-cancer individual (CV). The fold change of spot R211 was normalized by the total spot volume (TSV) for each individual gel.

**Table 2**

| **Type of cancer** | ***p*** | **Fold change** |
|---|---|---|
| Ta | 0.0226 | -3.05 |
| T1-low grade | 0.00565 | -2.82 |
| T1-high grade | 0.0212 | -3.84 |
| T2 | 0.0253 | -2.41 |

### II. Antibody development for the proteins found in the urine sample

Proteins found in the urine sample are excised from the gels and redisolved to be used for the immunization experiments. In case the amount of the identified proteins is not enough for immunization, recombinant proteins are generated.

Once polyclonal and monoclonal antibodies are made and tested for reactivity and sensitivity, the method generally involves preparation of a standard ("dose response") curve for the protein to be monitored. As indicated earlier, the stage of bladder cancer development may also be assessed by monitoring the concentrations of different stage-specific proteins in the sample.

### III. In-vitro method to detect TCC in a urine sample

Generally, the method involves the collection of a urine sample. The immunoassays are performed in the same body fluid.

### A. immunization Protocols

### 1. Polyclonal Antibodies

Polyclonal antisera can be raised against a given protein using standard methodologies, such as those disclosed in numerous texts available in the art and known to those generally skilled in the art. In this example, male New Zealand White rabbits are immunized with the protein preparations first in Freund's complete adjuvant (Gibco, Grand Island, N.Y.) and then every month with the protein with incomplete adjuvant for three months. Rabbit sera and sera from mice prior to fusion are used as polyclonal antisera and are shown by standard western blot technique to be reactive with the protein preparations.

### 2. Recombinant proteins generation

Proteins found in too low quantities for immunization are cloned and expressed in *E.coli* using the pET28b(+) cloning vector. Crude extracts are obtained as described previously (Boronat A., et al., J. Bacteriol. 1981, 147:181-85) and run on a SDS-PAGE gel. Recombinant proteins are excised from the gel and released from acrylamide. For the generation of antibodies against recombinant proteins, the released proteins are directly used to immunize rabbits as described above.

### B. Western blotting experiments

Protein samples (20 µg of total protein) are mixed with SDS-PAGE gel loading buffer supplemented with 5% β-mercaptoethanol and incubated at 100°C for 5 min, before being loaded on 6% polyacrylamide gel. Following electrophoresis proteins are transferred to nitrocellulose membranes. Duplicate gels are run and blotted. One membrane is proved with antibodies raised against one or more of the selected38 proteins of the invention (Santa Cruz Biotech. Inc., Santa Cruz, CA, USA.) while the second membrane is proved with antibody raised against actin (Amersham, Little Chalfont, UK) as a control for protein loading. Finally, membranes are hybridised with a secondary antibody conjugated with peroxidase (Amersham) and the chemoluminescent signal is detected using the ECL system (Amersham) with high performance chemiluminescence film (Hyperfilm ECL, Amersham).

### DESCRIPTION OF THE DRAWINGS

FIGURE 1: Bidimensional electrophoresis (2D) gel obtained from a urine sample and showing the four different studied areas A, K, R and S (FIGURE 1A), area A (FIGURE 1 B), area K (FIGURE 1 C), area R (FIGURE 1 D) and area S (FIGURE 1 E).
FIGURE 2: Spot R211 corresponding to BIEA in the R area of Bidimensional electrophoresis (2D) gel obtained from urine samples of a healthy individual (FIGURE 2A), of a patient suffering cancer at the Ta stage (FIGURE 2B), of a patient suffering cancer at the T1-low grade stage (T1-LG, FIGURE 2C), of a patient suffering cancer at the T1-high grade stage (T1-HG, FIGURE 2D) and of a patient suffering cancer at the T2 stage (FIGURE 2E).
FIGURE 3: Intensity of spot R211 in samples CV, Ta, T1-low grade (T1-LG), T1-high grade (T1-HG) and T2. The number of samples for each group is indicated in parentheses.
FIGURE 4: Robustness of the intensity measurement for spot R211 in different urine samples. This is expressed as the percentage of the gels that mantain the presence or absence of spot R211 in every gel analyzed. The number of samples for each group is indicated in parentheses.

## Claims

1. A non-invasive in vitro method that comprises:
a) the detection and/or quantification of one or more proteins selected from the set comprising ACY1 (SEQ ID 1), AK1A1 (SEQ ID 2), AMYC (SEQ ID 3), AMYP (SEQ ID 4), AMYS (SEQ ID 5), ANXA4 (SEQ ID 6), APOA1 (SEQ ID 7), BIEA (SEQ ID 8), BLVRB (SEQ ID 9), CATD (SEQ ID 10), CO3 (SEQ ID 11 or SEQ ID 12), CPGL1 (SEQ ID 13), ENOA (SEQ ID 14 or SEQ ID 15 or SEQ ID 16), FRIL (SEQ ID 17), GDIB (SEQ ID 18), GPX3 (SEQ ID 19), GSHB (SEQ ID 20), GSPT1 (SEQ ID 21), IDHC (SEQ ID 22), IGHG1 (SEQ ID 23), LMAN2 (SEQ ID 24), LY6G6E (SEQ ID 25), MASP2 (SEQ ID 26), NADC (SEQ ID 27), NAPSA (SEQ ID 28), PA2G4 (SEQ ID 29), PARK7 (SEQ ID 30), PCBP1 (SEQ ID 31), PDC61 (SEQ ID 32), PPAL (SEQ ID 33), PRDX2 (SEQ ID 34), PTD012 (SEQ ID 35), QPCT (SEQ ID 36), RETBP (SEQ ID 37), SBP1 (SEQ ID 38), STIP1 (SEQ ID 39), TALDO (SEQ ID 40) and WDR1 (SEQ ID 41) in a urine sample from an individual; and/or
b) the quantification of one or more ratios between the amount of one or more proteins selected from the set as defined in section a) in a urine sample from an individual, and one or more proteins independently selected from the group comprising constantly expressed proteins and differentially expressed proteins in the same urine sample.

2. Method according to claim 1 which is employed to detect the presence of transitional cell carcinoma of the bladder, to determine the stage or severity of this cancer, to assess the lack of disease after surgical resection, to establish the diagnosis and/or prognosis of this cancer and/or to monitor the effect of the treatment administered to an individual suffering from said cancer.

3. Method according to claim 1 or 2 in which the sample to be analyzed is obtained from an individual not previously diagnosed with transitional cell carcinoma of the bladder.

4. Method according to claim 1 or 2 in which the sample to be analyzed is obtained from an individual who has been previously diagnosed with transitional cell carcinoma of the bladder.

5. Method according to claim 1 or 2 in which the sample to be analyzed is obtained from an individual currently receiving treatment against transitional cell carcinoma of the bladder.

6. Method according to claim 1 or 2 **characterised in that** it comprises obtaining an extract of proteins of the sample.

7. Method according to claim 1 **characterised in that** the detection and/or quantification of proteins comprises a first step, in which the protein extract of the sample is placed in contact with a composition of one or more specific antibodies against one or more epitopes of the protein or proteins, and a second step, in which the complexes formed by the antibodies and the proteins are quantified.

8. Method according to claim 7 **characterised in that** said antibodies are of human origin, humanised or of non-human origin and selected from monoclonal or polyclonal antibodies, intact or recombinant fragments of antibodies, combibodies and Fab or scFv antibody fragments.

9. Method according to claim 7 or 8 **characterised in that** in the detection and/or quantification of the complexes formed by antibodies and proteins, the techniques used are selected from the group comprised by: western-blot, ELISA (Enzyme-Linked Immunosorbent assay), RIA (Radioimmunoassay), Competitive EIA (Competitive Enzyme lmmunoassay), DAS-ELISA (Double Antibody Sandwich-ELISA), immunocytochemical or immunohistochemical techniques, techniques based on the use of biochips or protein microarrays that include specific antibodies, assays based on the precipitation of colloidal gold in formats such as dipsticks; or by affinity chromatography techniques, ligand binding assays or lectin binding assays.

10. Method for diagnosing transitional cell carcinoma of the bladder, comprising the non-invasive in vitro method according to any one of claims 1-9.

11. Method according to any one of claims 1-10, wherein the method is used to monitor the efficacy of pharmacological or surgical treatment.

12. Method according to any one of claims 1-11, wherein the method is used to determine the progression of the disease when the same protein is compared from different samples obtained at different times.

13. Use of one or more peptide sequences derived from one or more proteins selected from selected from the set as defined in claim 1 to detect the presence of transitional cell carcinoma of the bladder, to determine the stage or severity of this cancer, to assess the lack of disease after surgical resection, to establish the diagnosis and/or prognosis of this cancer and/or to monitor the effect of the treatment administered to an individual suffering from said cancer.

14. Use of one or more nucleotides or peptide sequences derived from one or more proteins selected from the set as defined in claim 1, alone or in any combination, in methods to screen for, identify, develop and evaluate the efficiency of compounds to transitional cell carcinoma of the bladder.

15. The use as according to claims 13 or 14, wherein the proteins are present in urine.

16. A kit to perform a method as defined in claim 1 comprising any combination of antibodies that specifically recognises one or more of these proteins and a carrier in suitable packaging.

17. A kit according to claim 16 that is employed to detect the presence of transitional cell carcinoma of the bladder, to determine the stage or severity of this cancer, to assess the lack of disease after surgical resection, to establish the diagnosis and/or prognosis of this cancer and/or to monitor the effect of the treatment administered to an individual suffering from said cancer.

18. A kit according to claims 16 or 17 comprising a biochip.

19. A kit according to claim 18, wherein the biochip comprises antibodies for the detection of proteins selected from from the set as defined in claim 1, constantly expressed proteins and differentially expressed proteins.


SEQUENCE LISTING

<110>  LABORATORIOS SALVAT

<120>  NON-INVASIVE IN VITRO METHOD TO DETECT TRANSITIONAL CELL
       CARCINOMA OF THE BLADDER

<130>  110891

<160>  41

<170>  PatentIn version 3.3

<210>  1
<211>  408
<212>  PRT
<213>  Homo sapiens

<400>  1

<210>  2
<211>  324
<212>  PRT
<213>  Homo sapiens

<400>  2

Ala Ala Ser Cys Val Leu Leu His Thr Gly Gln Lys Met Pro Leu Ile
1               5                   10                  15

Gly Leu Gly Thr Trp Lys Ser Glu Pro Gly Gln Val Lys Ala Ala Val
            20                  25                  30

Lys Tyr Ala Leu Ser Val Gly Tyr Arg His Ile Asp Cys Ala Ala Ile
        35                  40                  45

Tyr Gly Asn Glu Pro Glu Ile Gly Glu Ala Leu Lys Glu Asp Val Gly
    50                  55                  60

Pro Gly Lys Ala Val Pro Arg Glu Glu Leu Phe Val Thr Ser Lys Leu
65                  70                  75                  80

Trp Asn Thr Lys His His Pro Glu Asp Val Glu Pro Ala Leu Arg Lys
                85                  90                  95

Thr Leu Ala Asp Leu Gln Leu Glu Tyr Leu Asp Leu Tyr Leu Met His
            100                 105                 110

Trp Pro Tyr Ala Phe Glu Arg Gly Asp Asn Pro Phe Pro Lys Asn Ala
        115                 120                 125

Asp Gly Thr Ile Cys Tyr Asp Ser Thr His Tyr Lys Glu Thr Trp Lys
    130                 135                 140

Ala Leu Glu Ala Leu Val Ala Lys Gly Leu Val Gln Ala Leu Gly Leu
145                 150                 155                 160

Ser Asn Phe Asn Ser Arg Gln Ile Asp Asp Ile Leu Ser Val Ala Ser
                165                 170                 175

Val Arg Pro Ala Val Leu Gln Val Glu Cys His Pro Tyr Leu Ala Gln
            180                 185                 190

Asn Glu Leu Ile Ala His Cys Gln Ala Arg Gly Leu Glu Val Thr Ala
        195                 200                 205

Tyr Ser Pro Leu Gly Ser Ser Asp Arg Ala Trp Arg Asp Pro Asp Glu
    210                 215                 220

Pro Val Leu Leu Glu Glu Pro Val Val Leu Ala Leu Ala Glu Lys Tyr
225                 230                 235                 240

Gly Arg Ser Pro Ala Gln Ile Leu Leu Arg Trp Gln Val Gln Arg Lys
                245                 250                 255

Val Ile Cys Ile Pro Lys Ser Ile Thr Pro Ser Arg Ile Leu Gln Asn
            260                 265                 270

Ile Lys Val Phe Asp Phe Thr Phe Ser Pro Glu Glu Met Lys Gln Leu
        275                 280                 285

Asn Ala Leu Asn Lys Asn Trp Arg Tyr Ile Val Pro Met Leu Thr Val
    290                 295                 300

Asp Gly Lys Arg Val Pro Arg Asp Ala Gly His Pro Leu Tyr Pro Phe
305                 310                 315                 320

Asn Asp Pro Tyr

<210>  3
<211>  511
<212>  PRT
<213>  Homo sapiens

<400>  3

Met Lys Phe Phe Leu Leu Leu Phe Thr Ile Gly Phe Cys Trp Ala Gln
1               5                   10                  15

Tyr Ser Pro Asn Thr Gln Gln Gly Arg Thr Ser Ile Val His Leu Phe
            20                  25                  30

Glu Trp Arg Trp Val Asp Ile Ala Leu Glu Cys Glu Arg Tyr Leu Ala
        35                  40                  45

Pro Lys Gly Phe Gly Gly Val Gln Val Ser Pro Pro Asn Glu Asn Val
    50                  55                  60

Ala Ile His Asn Pro Phe Arg Pro Trp Trp Glu Arg Tyr Gln Pro Val
65                  70                  75                  80

Ser Tyr Lys Leu Cys Thr Arg Ser Gly Asn Glu Asp Glu Phe Arg Asn
                85                  90                  95

Met Val Thr Arg Cys Asn Asn Val Gly Val Arg Ile Tyr Val Asp Ala
            100                 105                 110

Val Ile Asn His Met Ser Gly Asn Ala Val Ser Ala Gly Thr Ser Ser
        115                 120                 125

Thr Cys Gly Ser Tyr Phe Asn Pro Gly Ser Arg Asp Phe Pro Ala Val
    130                 135                 140

Pro Tyr Ser Gly Trp Asp Phe Asn Asp Gly Lys Cys Lys Thr Gly Ser
145                 150                 155                 160

Gly Asp Ile Glu Asn Tyr Asn Asp Ala Thr Gln Val Arg Asp Cys Arg
                165                 170                 175

Leu Val Gly Leu Leu Asp Leu Ala Leu Glu Lys Asp Tyr Val Arg Ser
            180                 185                 190

Lys Ile Ala Glu Tyr Met Asn His Leu Ile Asp Ile Gly Val Ala Gly
        195                 200                 205

Phe Arg Leu Asp Ala Ser Lys His Met Trp Pro Gly Asp Ile Lys Ala
    210                 215                 220

Ile Leu Asp Lys Leu His Asn Leu Asn Ser Asn Trp Phe Pro Ala Gly
225                 230                 235                 240

Ser Lys Pro Phe Ile Tyr Gln Glu Val Ile Asp Leu Gly Gly Glu Pro
                245                 250                 255

Ile Lys Ser Ser Asp Tyr Phe Gly Asn Gly Arg Val Thr Glu Phe Lys
            260                 265                 270

Tyr Gly Ala Lys Leu Gly Thr Val Ile Arg Lys Trp Asn Gly Glu Lys
        275                 280                 285

Met Ser Tyr Leu Lys Asn Trp Gly Glu Gly Trp Gly Phe Met Pro Ser
    290                 295                 300

Asp Arg Ala Leu Val Phe Val Asp Asn His Asp Asn Gln Arg Gly His
305                 310                 315                 320

Gly Ala Gly Gly Ala Ser Ile Leu Thr Phe Trp Asp Ala Arg Leu Tyr
                325                 330                 335

Lys Met Ala Val Gly Phe Met Leu Ala His Pro Tyr Gly Phe Thr Arg
            340                 345                 350

Val Met Ser Ser Tyr Arg Trp Pro Arg Gln Phe Gln Asn Gly Asn Asp
        355                 360                 365

Val Asn Asp Trp Val Gly Pro Pro Asn Asn Asn Gly Val Ile Lys Glu
    370                 375                 380

Val Thr Ile Asn Pro Asp Thr Thr Cys Gly Asn Asp Trp Val Cys Glu
385                 390                 395                 400

His Arg Trp Arg Gln Ile Arg Asn Met Val Asn Phe Arg Asn Val Val
                405                 410                 415

Asp Gly Gln Pro Phe Thr Asn Trp Tyr Asp Asn Gly Ser Asn Gln Val
            420                 425                 430

Ala Phe Gly Arg Gly Asn Arg Gly Phe Ile Val Phe Asn Asn Asp Asp
        435                 440                 445

Trp Thr Phe Ser Leu Thr Leu Gln Thr Gly Leu Pro Ala Gly Thr Tyr
    450                 455                 460

Cys Asp Val Ile Ser Gly Asp Lys Ile Asn Gly Asn Cys Thr Gly Ile
465                 470                 475                 480

Lys Ile Tyr Val Ser Asp Asp Gly Lys Ala His Phe Ser Ile Ser Asn
                485                 490                 495

Ser Ala Glu Asp Pro Phe Ile Ala Ile His Ala Glu Ser Lys Leu
            500                 505                 510

<210>  4
<211>  511
<212>  PRT
<213>  Homo sapiens

<400>  4

Met Lys Phe Phe Leu Leu Leu Phe Thr Ile Gly Phe Cys Trp Ala Gln
1               5                   10                  15

Tyr Ser Pro Asn Thr Gln Gln Gly Arg Thr Ser Ile Val His Leu Phe
            20                  25                  30

Glu Trp Arg Trp Val Asp Ile Ala Leu Glu Cys Glu Arg Tyr Leu Ala
        35                  40                  45

Pro Lys Gly Phe Gly Gly Val Gln Val Ser Pro Pro Asn Glu Asn Val
    50                  55                  60

Ala Ile His Asn Pro Phe Arg Pro Trp Trp Glu Arg Tyr Gln Pro Val
65                  70                  75                  80

Ser Tyr Lys Leu Cys Thr Arg Ser Gly Asn Glu Asp Glu Phe Arg Asn
                85                  90                  95

Met Val Thr Arg Cys Asn Asn Val Gly Val Arg Ile Tyr Val Asp Ala
            100                 105                 110

Val Ile Asn His Met Ser Gly Asn Ala Val Ser Ala Gly Thr Ser Ser
        115                 120                 125

Thr Cys Gly Ser Tyr Phe Asn Pro Gly Ser Arg Asp Phe Pro Ala Val
    130                 135                 140

Pro Tyr Ser Gly Trp Asp Phe Asn Asp Gly Lys Cys Lys Thr Gly Ser
145                 150                 155                 160

Gly Asp Ile Glu Asn Tyr Asn Asp Ala Thr Gln Val Arg Asp Cys Arg
                165                 170                 175

Leu Val Gly Leu Leu Asp Leu Ala Leu Glu Lys Asp Tyr Val Arg Ser
            180                 185                 190

Lys Ile Ala Glu Tyr Met Asn His Leu Ile Asp Ile Gly Val Ala Gly
        195                 200                 205

Phe Arg Leu Asp Ala Ser Lys His Met Trp Pro Gly Asp Ile Lys Ala
    210                 215                 220

Ile Leu Asp Lys Leu His Asn Leu Asn Ser Asn Trp Phe Pro Ala Gly

225                 230                 235                 240

Ser Lys Pro Phe Ile Tyr Gln Glu Val Ile Asp Leu Gly Gly Glu Pro
                245                 250                 255

Ile Lys Ser Ser Asp Tyr Phe Gly Asn Gly Arg Val Thr Glu Phe Lys
            260                 265                 270

Tyr Gly Ala Lys Leu Gly Thr Val Ile Arg Lys Trp Asn Gly Glu Lys
        275                 280                 285

Met Ser Tyr Leu Lys Asn Trp Gly Glu Gly Trp Gly Phe Met Pro Ser
    290                 295                 300

Asp Arg Ala Leu Val Phe Val Asp Asn His Asp Asn Gln Arg Gly His
305                 310                 315                 320

Gly Ala Gly Gly Ala Ser Ile Leu Thr Phe Trp Asp Ala Arg Leu Tyr
                325                 330                 335

Lys Met Ala Val Gly Phe Met Leu Ala His Pro Tyr Gly Phe Thr Arg
            340                 345                 350

Val Met Ser Ser Tyr Arg Trp Pro Arg Gln Phe Gln Asn Gly Asn Asp
        355                 360                 365

Val Asn Asp Trp Val Gly Pro Pro Asn Asn Asn Gly Val Ile Lys Glu
    370                 375                 380

Val Thr Ile Asn Pro Asp Thr Thr Cys Gly Asn Asp Trp Val Cys Glu
385                 390                 395                 400

His Arg Trp Arg Gln Ile Arg Asn Met Val Asn Phe Arg Asn Val Val
                405                 410                 415

Asp Gly Gln Pro Phe Thr Asn Trp Tyr Asp Asn Gly Ser Asn Gln Val
            420                 425                 430

Ala Phe Gly Arg Gly Asn Arg Gly Phe Ile Val Phe Asn Asn Asp Asp
        435                 440                 445

Trp Thr Phe Ser Leu Thr Leu Gln Thr Gly Leu Pro Ala Gly Thr Tyr
    450                 455                 460

Cys Asp Val Ile Ser Gly Asp Lys Ile Asn Gly Asn Cys Thr Gly Ile
465                 470                 475                 480

Lys Ile Tyr Val Ser Asp Asp Gly Lys Ala His Phe Ser Ile Ser Asn
                485                 490                 495

Ser Ala Glu Asp Pro Phe Ile Ala Ile His Ala Glu Ser Lys Leu
            500                 505                 510

<210>  5
<211>  496
<212>  PRT
<213>  Homo sapiens

<220>
<221>  misc_feature
<222>  (1)..(1)
<223>  Xaa can be any naturally occurring amino acid

<400>  5

Xaa Tyr Ser Ser Asn Thr Gln Gln Gly Arg Thr Ser Ile Val His Leu
1               5                   10                  15

Phe Glu Trp Arg Trp Val Asp Ile Ala Leu Glu Cys Glu Arg Tyr Leu
            20                  25                  30

Ala Pro Lys Gly Phe Gly Gly Val Gln Val Ser Pro Pro Asn Glu Asn
        35                  40                  45

Val Ala Ile His Asn Pro Phe Arg Pro Trp Trp Glu Arg Tyr Gln Pro
    50                  55                  60

Val Ser Tyr Lys Leu Cys Thr Arg Ser Gly Asn Glu Asp Glu Phe Arg
65                  70                  75                  80

Asn Met Val Thr Arg Cys Asn Asn Val Gly Val Arg Ile Tyr Val Asp
                85                  90                  95

Ala Val Ile Asn His Met Cys Gly Asn Ala Val Ser Ala Gly Thr Ser
            100                 105                 110

Ser Thr Cys Gly Ser Tyr Phe Asn Pro Gly Ser Arg Asp Phe Pro Ala
        115                 120                 125

Val Pro Tyr Ser Gly Trp Asp Phe Asn Asp Gly Lys Cys Lys Thr Gly
    130                 135                 140

Ser Gly Asp Ile Glu Asn Tyr Asn Asp Ala Thr Gln Val Arg Asp Cys
145                 150                 155                 160

Arg Leu Ser Gly Leu Leu Asp Leu Ala Leu Gly Lys Asp Tyr Val Arg
                165                 170                 175

Ser Lys Ile Ala Glu Tyr Met Asn His Leu Ile Asp Ile Gly Val Ala
            180                 185                 190

Gly Phe Arg Ile Asp Ala Ser Lys His Met Trp Pro Gly Asp Ile Lys
        195                 200                 205

Ala Ile Leu Asp Lys Leu His Asn Leu Asn Ser Asn Trp Phe Pro Glu
    210                 215                 220

Gly Ser Lys Pro Phe Ile Tyr Gln Glu Val Ile Asp Leu Gly Gly Glu
225                 230                 235                 240

Pro Ile Lys Ser Ser Asp Tyr Phe Gly Asn Gly Arg Val Thr Glu Phe
                245                 250                 255

Lys Tyr Gly Ala Lys Leu Gly Thr Val Ile Arg Lys Trp Asn Gly Glu
            260                 265                 270

Lys Met Ser Tyr Leu Lys Asn Trp Gly Glu Gly Trp Gly Phe Met Pro
        275                 280                 285

Ser Asp Arg Ala Leu Val Phe Val Asp Asn His Asp Asn Gln Arg Gly
    290                 295                 300

His Gly Ala Gly Gly Ala Ser Ile Leu Thr Phe Trp Asp Ala Arg Leu
305                 310                 315                 320

Tyr Lys Met Ala Val Gly Phe Met Leu Ala His Pro Tyr Gly Phe Thr
                325                 330                 335

Arg Val Met Ser Ser Tyr Arg Trp Pro Arg Tyr Phe Glu Asn Gly Lys
            340                 345                 350

Asp Val Asn Asp Trp Val Gly Pro Pro Asn Asp Asn Gly Val Thr Lys
        355                 360                 365

Glu Val Thr Ile Asn Pro Asp Thr Thr Cys Gly Asn Asp Trp Val Cys
    370                 375                 380

Glu His Arg Trp Arg Gln Ile Arg Asn Met Val Asn Phe Arg Asn Val
385                 390                 395                 400

Val Asp Gly Gln Pro Phe Thr Asn Trp Tyr Asp Asn Gly Ser Asn Gln
                405                 410                 415

Val Ala Phe Gly Arg Gly Asn Arg Gly Phe Ile Val Phe Asn Asn Asp
            420                 425                 430

Asp Trp Thr Phe Ser Leu Thr Leu Gln Thr Gly Leu Pro Ala Gly Thr
        435                 440                 445

Tyr Cys Asp Val Ile Ser Gly Asp Lys Ile Asn Gly Asn Cys Thr Gly
    450                 455                 460

Ile Lys Ile Tyr Val Ser Asp Asp Gly Lys Ala His Phe Ser Ile Ser
465                 470                 475                 480

Asn Ser Ala Glu Asp Pro Phe Ile Ala Ile His Ala Glu Ser Lys Leu
                485                 490                 495

<210>  6
<211>  319
<212>  PRT
<213>  Homo sapiens

<400>  6

Met Ala Thr Lys Gly Gly Thr Val Lys Ala Ala Ser Gly Phe Asn Ala
1               5                   10                  15

Met Glu Asp Ala Gln Thr Leu Arg Lys Ala Met Lys Gly Leu Gly Thr
            20                  25                  30

Asp Glu Asp Ala Ile Ile Ser Val Leu Ala Tyr Arg Asn Thr Ala Gln
        35                  40                  45

Arg Gln Glu Ile Arg Thr Ala Tyr Lys Ser Thr Ile Gly Arg Asp Leu
    50                  55                  60

Ile Asp Asp Leu Lys Ser Glu Leu Ser Gly Asn Phe Glu Gln Val Ile
65                  70                  75                  80

Val Gly Met Met Thr Pro Thr Val Leu Tyr Asp Val Gln Glu Leu Arg
                85                  90                  95

Arg Ala Met Lys Gly Ala Gly Thr Asp Glu Gly Cys Leu Ile Glu Ile
            100                 105                 110

Leu Ala Ser Arg Thr Pro Glu Glu Ile Arg Arg Ile Ser Gln Thr Tyr
        115                 120                 125

Gln Gln Gln Tyr Gly Arg Ser Leu Glu Asp Asp Ile Arg Ser Asp Thr
    130                 135                 140

Ser Phe Met Phe Gln Arg Val Leu Val Ser Leu Ser Ala Gly Gly Arg
145                 150                 155                 160

Asp Glu Gly Asn Tyr Leu Asp Asp Ala Leu Val Arg Gln Asp Ala Gln
                165                 170                 175

Asp Leu Tyr Glu Ala Gly Glu Lys Lys Trp Gly Thr Asp Glu Val Lys
            180                 185                 190

Phe Leu Thr Val Leu Cys Ser Arg Asn Arg Asn His Leu Leu His Val
        195                 200                 205

Phe Asp Glu Tyr Lys Arg Ile Ser Gln Lys Asp Ile Glu Gln Ser Ile
    210                 215                 220

Lys Ser Glu Thr Ser Gly Ser Phe Glu Asp Ala Leu Leu Ala Ile Val
225                 230                 235                 240

Lys Cys Met Arg Asn Lys Ser Ala Tyr Phe Ala Glu Lys Leu Tyr Lys

245                 250                 255

Ser Met Lys Gly Leu Gly Thr Asp Asp Asn Thr Leu Ile Arg Val Met
            260                 265                 270

Val Ser Arg Ala Glu Ile Asp Met Leu Asp Ile Arg Ala His Phe Lys
        275                 280                 285

Arg Leu Tyr Gly Lys Ser Leu Tyr Ser Phe Ile Lys Gly Asp Thr Ser
    290                 295                 300

Gly Asp Tyr Arg Lys Val Leu Leu Val Leu Cys Gly Gly Asp Asp
305                 310                 315

<210>  7
<211>  249
<212>  PRT
<213>  Homo sapiens

<400>  7

Arg His Phe Trp Gln Gln Asp Glu Pro Pro Gln Ser Pro Trp Asp Arg
1               5                   10                  15

Val Lys Asp Leu Ala Thr Val Tyr Val Asp Val Leu Lys Asp Ser Gly
            20                  25                  30

Arg Asp Tyr Val Ser Gln Phe Glu Gly Ser Ala Leu Gly Lys Gln Leu
        35                  40                  45

Asn Leu Lys Leu Leu Asp Asn Trp Asp Ser Val Thr Ser Thr Phe Ser
    50                  55                  60

Lys Leu Arg Glu Gln Leu Gly Pro Val Thr Gln Glu Phe Trp Asp Asn
65                  70                  75                  80

Leu Glu Lys Glu Thr Glu Gly Leu Arg Gln Glu Met Ser Lys Asp Leu
                85                  90                  95

Glu Glu Val Lys Ala Lys Val Gln Pro Tyr Leu Asp Asp Phe Gln Lys
            100                 105                 110

Lys Trp Gln Glu Glu Met Glu Leu Tyr Arg Gln Lys Val Glu Pro Leu
        115                 120                 125

Arg Ala Glu Leu Gln Glu Gly Ala Arg Gln Lys Leu His Glu Leu Gln
    130                 135                 140

Glu Lys Leu Ser Pro Leu Gly Glu Glu Met Arg Asp Arg Ala Arg Ala
145                 150                 155                 160

His Val Asp Ala Leu Arg Thr His Leu Ala Pro Tyr Ser Asp Glu Leu
                165                 170                 175

Arg Gln Arg Leu Ala Ala Arg Leu Glu Ala Leu Lys Glu Asn Gly Gly
            180                 185                 190

Ala Arg Leu Ala Glu Tyr His Ala Lys Ala Thr Glu His Leu Ser Thr
        195                 200                 205

Leu Ser Glu Lys Ala Lys Pro Ala Leu Glu Asp Leu Arg Gln Gly Leu
    210                 215                 220

Leu Pro Val Leu Glu Ser Phe Lys Val Ser Phe Leu Ser Ala Leu Glu
225                 230                 235                 240

Glu Tyr Thr Lys Lys Leu Asn Thr Gln
                245

<210>  8
<211>  296
<212>  PRT
<213>  Homo sapiens

<400>  8

Met Asn Thr Glu Pro Glu Arg Lys Phe Gly Val Val Val Val Gly Val
1               5                   10                  15

Gly Arg Ala Gly Ser Val Arg Met Arg Asp Leu Arg Asn Pro His Pro
            20                  25                  30

Ser Ser Ala Phe Leu Asn Leu Ile Gly Phe Val Ser Arg Arg Glu Leu
        35                  40                  45

Gly Ser Ile Asp Gly Val Gln Gln Ile Ser Leu Glu Asp Ala Leu Ser
    50                  55                  60

Ser Gln Glu Val Glu Val Ala Tyr Ile Cys Ser Glu Ser Ser Ser His
65                  70                  75                  80

Glu Asp Tyr Ile Arg Gln Phe Leu Asn Ala Gly Lys His Val Leu Val
                85                  90                  95

Glu Tyr Pro Met Thr Leu Ser Leu Ala Ala Ala Gln Glu Leu Trp Glu
            100                 105                 110

Leu Ala Glu Gln Lys Gly Lys Val Leu His Glu Glu His Val Glu Leu
        115                 120                 125

Leu Met Glu Glu Phe Ala Phe Leu Lys Lys Glu Val Val Gly Lys Asp
    130                 135                 140

Leu Leu Lys Gly Ser Leu Leu Phe Thr Ala Gly Pro Leu Glu Glu Glu
145                 150                 155                 160

Arg Phe Gly Phe Pro Ala Phe Ser Gly Ile Ser Arg Leu Thr Trp Leu

165                 170                 175

Val Ser Leu Phe Gly Glu Leu Ser Leu Val Ser Ala Thr Leu Glu Glu
            180                 185                 190

Arg Lys Glu Asp Gln Tyr Met Lys Met Thr Val Cys Leu Glu Thr Glu
        195                 200                 205

Lys Lys Ser Pro Leu Ser Trp Ile Glu Glu Lys Gly Pro Gly Leu Lys
    210                 215                 220

Arg Asn Arg Tyr Leu Ser Phe His Phe Lys Ser Gly Ser Leu Glu Asn
225                 230                 235                 240

Val Pro Asn Val Gly Val Asn Lys Asn Ile Phe Leu Lys Asp Gln Asn
                245                 250                 255

Ile Phe Val Gln Lys Leu Leu Gly Gln Phe Ser Glu Lys Glu Leu Ala
            260                 265                 270

Ala Glu Lys Lys Arg Ile Leu His Cys Leu Gly Leu Ala Glu Glu Ile
        275                 280                 285

Gln Lys Tyr Cys Cys Ser Arg Lys
    290                 295

<210>  9
<211>  206
<212>  PRT
<213>  Homo sapiens

<400>  9

Met Ala Val Lys Lys Ile Ala Ile Phe Gly Ala Thr Gly Gln Thr Gly
1               5                   10                  15

Leu Thr Thr Leu Ala Gln Ala Val Gln Ala Gly Tyr Glu Val Thr Val
            20                  25                  30

Leu Val Arg Asp Ser Ser Arg Leu Pro Ser Glu Gly Pro Arg Pro Ala
        35                  40                  45

His Val Val Val Gly Asp Val Leu Gln Ala Ala Asp Val Asp Lys Thr
    50                  55                  60

Val Ala Gly Gln Asp Ala Val Ile Val Leu Leu Gly Thr Arg Asn Asp
65                  70                  75                  80

Leu Ser Pro Thr Thr Val Met Ser Glu Gly Ala Arg Asn Ile Val Ala
                85                  90                  95

Ala Met Lys Ala His Gly Val Asp Lys Val Val Ala Cys Thr Ser Ala
            100                 105                 110

Phe Leu Leu Trp Asp Pro Thr Lys Val Pro Pro Arg Leu Gln Ala Val
        115                 120                 125

Thr Asp Asp His Ile Arg Met His Lys Val Leu Arg Glu Ser Gly Leu
    130                 135                 140

Lys Tyr Val Ala Val Met Pro Pro His Ile Gly Asp Gln Pro Leu Thr
145                 150                 155                 160

Gly Ala Tyr Thr Val Thr Leu Asp Gly Arg Gly Pro Ser Arg Val Ile
                165                 170                 175

Ser Lys His Asp Leu Gly His Phe Met Leu Arg Cys Leu Thr Thr Asp
            180                 185                 190

Glu Tyr Asp Gly His Ser Thr Tyr Pro Ser His Gln Tyr Gln
        195                 200                 205

<210>  10
<211>  413
<212>  PRT
<213>  Artificial

<220>
<223>  Conceptual translation

<400>  10

Met Gln Pro Ser Ser Leu Leu Pro Leu Ala Leu Cys Leu Leu Ala Ala
1               5                   10                  15

Pro Ala Ser Ala Leu Val Arg Ile Pro Leu His Lys Phe Thr Ser Ile
            20                  25                  30

Arg Arg Thr Met Ser Glu Val Gly Gly Ser Val Glu Asp Leu Ile Ala
        35                  40                  45

Lys Gly Pro Val Ser Lys Tyr Ser Gln Ala Val Pro Ala Val Thr Glu
    50                  55                  60

Gly Pro Ile Pro Glu Val Leu Lys Asn Tyr Met Asp Ala Gln Tyr Tyr
65                  70                  75                  80

Gly Glu Ile Gly Ile Gly Thr Pro Pro Gln Cys Phe Thr Val Val Phe
                85                  90                  95

Asp Thr Gly Ser Ser Asn Leu Trp Val Pro Ser Ile His Cys Lys Leu
            100                 105                 110

Leu Asp Ile Ala Cys Trp Ile His His Lys Tyr Asn Ser Asp Lys Ser
        115                 120                 125

Ser Thr Tyr Val Lys Asn Gly Thr Ser Phe Asp Ile His Tyr Gly Ser
    130                 135                 140

Gly Ser Leu Ser Gly Tyr Leu Ser Gln Asp Thr Val Ser Val Pro Cys
145                 150                 155                 160

Gln Ser Ala Ser Ser Ala Ser Ala Leu Gly Gly Val Lys Val Glu Arg
                165                 170                 175

Gln Val Phe Gly Glu Ala Thr Lys Gln Pro Gly Ile Thr Phe Ile Ala
            180                 185                 190

Ala Lys Phe Asp Gly Ile Leu Gly Met Ala Tyr Pro Arg Ile Ser Val
        195                 200                 205

Asn Asn Val Leu Pro Val Phe Asp Asn Leu Met Gln Gln Lys Leu Val
    210                 215                 220

Asp Gln Asn Ile Phe Ser Phe Tyr Leu Ser Arg Asp Pro Asp Ala Gln
225                 230                 235                 240

Pro Gly Gly Glu Leu Met Leu Gly Gly Thr Asp Ser Lys Tyr Tyr Lys
                245                 250                 255

Gly Ser Leu Ser Tyr Leu Asn Val Thr Arg Lys Ala Tyr Trp Gln Val
            260                 265                 270

His Leu Asp Gln Val Glu Val Ala Ser Gly Leu Thr Leu Cys Lys Glu
        275                 280                 285

Gly Cys Glu Ala Ile Val Asp Thr Gly Thr Ser Leu Met Val Gly Pro
    290                 295                 300

Val Asp Glu Val Arg Glu Leu Gln Lys Ala Ile Gly Ala Val Pro Leu
305                 310                 315                 320

Ile Gln Gly Glu Tyr Met Ile Pro Cys Glu Lys Val Ser Thr Leu Pro
                325                 330                 335

Ala Ile Thr Leu Lys Leu Gly Gly Lys Gly Tyr Lys Leu Ser Pro Glu
            340                 345                 350

Asp Tyr Thr Leu Lys Val Ser Gln Ala Gly Lys Thr Leu Cys Leu Ser
        355                 360                 365

Gly Phe Met Gly Met Asp Ile Pro Pro Pro Ser Gly Pro Leu Trp Ile
    370                 375                 380

Leu Gly Asp Val Phe Ile Gly Arg Tyr Tyr Thr Val Phe Asp Arg Asp
385                 390                 395                 400

Asn Asn Arg Val Gly Phe Ala Glu Ala Ala Arg Leu Leu
                405                 410

<210>  11
<211>  1663
<212>  PRT
<213>  Homo sapiens

<400>  11

Met Gly Pro Thr Ser Gly Pro Ser Leu Leu Leu Leu Leu Leu Thr His
1               5                   10                  15

Leu Pro Leu Ala Leu Gly Ser Pro Met Tyr Ser Ile Ile Thr Pro Asn
            20                  25                  30

Ile Leu Arg Leu Glu Ser Glu Glu Thr Met Val Leu Glu Ala His Asp
        35                  40                  45

Ala Gln Gly Asp Val Pro Val Thr Val Thr Val His Asp Phe Pro Gly
    50                  55                  60

Lys Lys Leu Val Leu Ser Ser Glu Lys Thr Val Leu Thr Pro Ala Thr
65                  70                  75                  80

Asn His Met Gly Asn Val Thr Phe Thr Ile Pro Ala Asn Arg Glu Phe
                85                  90                  95

Lys Ser Glu Lys Gly Arg Asn Lys Phe Val Thr Val Gln Ala Thr Phe
            100                 105                 110

Gly Thr Gln Val Val Glu Lys Val Val Leu Val Ser Leu Gln Ser Gly
        115                 120                 125

Tyr Leu Phe Ile Gln Thr Asp Lys Thr Ile Tyr Thr Pro Gly Ser Thr
    130                 135                 140

Val Leu Tyr Arg Ile Phe Thr Val Asn His Lys Leu Leu Pro Val Gly
145                 150                 155                 160

Arg Thr Val Met Val Asn Ile Glu Asn Pro Glu Gly Ile Pro Val Lys
                165                 170                 175

Gln Asp Ser Leu Ser Ser Gln Asn Gln Leu Gly Val Leu Pro Leu Ser
            180                 185                 190

Trp Asp Ile Pro Glu Leu Val Asn Met Gly Gln Trp Lys Ile Arg Ala
        195                 200                 205

Tyr Tyr Glu Asn Ser Pro Gln Gln Val Phe Ser Thr Glu Phe Glu Val
    210                 215                 220

Lys Glu Tyr Val Leu Pro Ser Phe Glu Val Ile Val Glu Pro Thr Glu
225                 230                 235                 240

Lys Phe Tyr Tyr Ile Tyr Asn Glu Lys Gly Leu Glu Val Thr Ile Thr

245                 250                 255

Ala Arg Phe Leu Tyr Gly Lys Lys Val Glu Gly Thr Ala Phe Val Ile
            260                 265                 270

Phe Gly Ile Gln Asp Gly Glu Gln Arg Ile Ser Leu Pro Glu Ser Leu
        275                 280                 285

Lys Arg Ile Pro Ile Glu Asp Gly Ser Gly Glu Val Val Leu Ser Arg
    290                 295                 300

Lys Val Leu Leu Asp Gly Val Gln Asn Leu Arg Ala Glu Asp Leu Val
305                 310                 315                 320

Gly Lys Ser Leu Tyr Val Ser Ala Thr Val Ile Leu His Ser Gly Ser
                325                 330                 335

Asp Met Val Gln Ala Glu Arg Ser Gly Ile Pro Ile Val Thr Ser Pro
            340                 345                 350

Tyr Gln Ile His Phe Thr Lys Thr Pro Lys Tyr Phe Lys Pro Gly Met
        355                 360                 365

Pro Phe Asp Leu Met Val Phe Val Thr Asn Pro Asp Gly Ser Pro Ala
    370                 375                 380

Tyr Arg Val Pro Val Ala Val Gln Gly Glu Asp Thr Val Gln Ser Leu
385                 390                 395                 400

Thr Gln Gly Asp Gly Val Ala Lys Leu Ser Ile Asn Thr His Pro Ser
                405                 410                 415

Gln Lys Pro Leu Ser Ile Thr Val Arg Thr Lys Lys Gln Glu Leu Ser
            420                 425                 430

Glu Ala Glu Gln Ala Thr Arg Thr Met Gln Ala Leu Pro Tyr Ser Thr
        435                 440                 445

Val Gly Asn Ser Asn Asn Tyr Leu His Leu Ser Val Leu Arg Thr Glu
    450                 455                 460

Leu Arg Pro Gly Glu Thr Leu Asn Val Asn Phe Leu Leu Arg Met Asp
465                 470                 475                 480

Arg Ala His Glu Ala Lys Ile Arg Tyr Tyr Thr Tyr Leu Ile Met Asn
                485                 490                 495

Lys Gly Arg Leu Leu Lys Ala Gly Arg Gln Val Arg Glu Pro Gly Gln
            500                 505                 510

Asp Leu Val Val Leu Pro Leu Ser Ile Thr Thr Asp Phe Ile Pro Ser
        515                 520                 525

Phe Arg Leu Val Ala Tyr Tyr Thr Leu Ile Gly Ala Ser Gly Gln Arg
    530                 535                 540

Glu Val Val Ala Asp Ser Val Trp Val Asp Val Lys Asp Ser Cys Val
545                 550                 555                 560

Gly Ser Leu Val Val Lys Ser Gly Gln Ser Glu Asp Arg Gln Pro Val
                565                 570                 575

Pro Gly Gln Gln Met Thr Leu Lys Ile Glu Gly Asp His Gly Ala Arg
            580                 585                 590

Val Val Leu Val Ala Val Asp Lys Gly Val Phe Val Leu Asn Lys Lys
        595                 600                 605

Asn Lys Leu Thr Gln Ser Lys Ile Trp Asp Val Val Glu Lys Ala Asp
    610                 615                 620

Ile Gly Cys Thr Pro Gly Ser Gly Lys Asp Tyr Ala Gly Val Phe Ser
625                 630                 635                 640

Asp Ala Gly Leu Thr Phe Thr Ser Ser Ser Gly Gln Gln Thr Ala Gln
                645                 650                 655

Arg Ala Glu Leu Gln Cys Pro Gln Pro Ala Ala Arg Arg Arg Arg Ser
            660                 665                 670

Val Gln Leu Thr Glu Lys Arg Met Asp Lys Val Gly Lys Tyr Pro Lys
        675                 680                 685

Glu Leu Arg Lys Cys Cys Glu Asp Gly Met Arg Glu Asn Pro Met Arg
    690                 695                 700

Phe Ser Cys Gln Arg Arg Thr Arg Phe Ile Ser Leu Gly Glu Ala Cys
705                 710                 715                 720

Lys Lys Val Phe Leu Asp Cys Cys Asn Tyr Ile Thr Glu Leu Arg Arg
                725                 730                 735

Gln His Ala Arg Ala Ser His Leu Gly Leu Ala Arg Ser Asn Leu Asp
            740                 745                 750

Glu Asp Ile Ile Ala Glu Glu Asn Ile Val Ser Arg Ser Glu Phe Pro
        755                 760                 765

Glu Ser Trp Leu Trp Asn Val Glu Asp Leu Lys Glu Pro Pro Lys Asn
    770                 775                 780

Gly Ile Ser Thr Lys Leu Met Asn Ile Phe Leu Lys Asp Ser Ile Thr
785                 790                 795                 800

Thr Trp Glu Ile Leu Ala Val Ser Met Ser Asp Lys Lys Gly Ile Cys
                805                 810                 815

Val Ala Asp Pro Phe Glu Val Thr Val Met Gln Asp Phe Phe Ile Asp
            820                 825                 830

Leu Arg Leu Pro Tyr Ser Val Val Arg Asn Glu Gln Val Glu Ile Arg
        835                 840                 845

Ala Val Leu Tyr Asn Tyr Arg Gln Asn Gln Glu Leu Lys Val Arg Val
    850                 855                 860

Glu Leu Leu His Asn Pro Ala Phe Cys Ser Leu Ala Thr Thr Lys Arg
865                 870                 875                 880

Arg His Gln Gln Thr Val Thr Ile Pro Pro Lys Ser Ser Leu Ser Val
                885                 890                 895

Pro Tyr Val Ile Val Pro Leu Lys Thr Gly Leu Gln Glu Val Glu Val
            900                 905                 910

Lys Ala Ala Val Tyr His His Phe Ile Ser Asp Gly Val Arg Lys Ser
        915                 920                 925

Leu Lys Val Val Pro Glu Gly Ile Arg Met Asn Lys Thr Val Ala Val
    930                 935                 940

Arg Thr Leu Asp Pro Glu Arg Leu Gly Arg Glu Gly Val Gln Lys Glu
945                 950                 955                 960

Asp Ile Pro Pro Ala Asp Leu Ser Asp Gln Val Pro Asp Thr Glu Ser
                965                 970                 975

Glu Thr Arg Ile Leu Leu Gln Gly Thr Pro Val Ala Gln Met Thr Glu
            980                 985                 990

Asp Ala Val Asp Ala Glu Arg Leu  Lys His Leu Ile Val  Thr Pro Ser
        995                 1000                 1005

Gly Cys  Gly Glu Gln Asn Met  Ile Gly Met Thr Pro  Thr Val Ile
    1010                 1015                 1020

Ala Val  His Tyr Leu Asp Glu  Thr Glu Gln Trp Glu  Lys Phe Gly
    1025                 1030                 1035

Leu Glu  Lys Arg Gln Gly Ala  Leu Glu Leu Ile Lys  Lys Gly Tyr
    1040                 1045                 1050

Thr Gln  Gln Leu Ala Phe Arg  Gln Pro Ser Ser Ala  Phe Ala Ala
    1055                 1060                 1065

Phe Val Lys Arg Ala Pro Ser Thr Trp Leu Thr Ala Tyr Val Val
    1070                1075                1080

Lys Val Phe Ser Leu Ala Val Asn Leu Ile Ala Ile Asp Ser Gln
    1085                1090                1095

Val Leu Cys Gly Ala Val Lys Trp Leu Ile Leu Glu Lys Gln Lys
    1100                1105                1110

Pro Asp Gly Val Phe Gln Glu Asp Ala Pro Val Ile His Gln Glu
    1115                1120                1125

Met Ile Gly Gly Leu Arg Asn Asn Asn Glu Lys Asp Met Ala Leu
    1130                1135                1140

Thr Ala Phe Val Leu Ile Ser Leu Gln Glu Ala Lys Asp Ile Cys
    1145                1150                1155

Glu Glu Gln Val Asn Ser Leu Pro Gly Ser Ile Thr Lys Ala Gly
    1160                1165                1170

Asp Phe Leu Glu Ala Asn Tyr Met Asn Leu Gln Arg Ser Tyr Thr
    1175                1180                1185

Val Ala Ile Ala Gly Tyr Ala Leu Ala Gln Met Gly Arg Leu Lys
    1190                1195                1200

Gly Pro Leu Leu Asn Lys Phe Leu Thr Thr Ala Lys Asp Lys Asn
    1205                1210                1215

Arg Trp Glu Asp Pro Gly Lys Gln Leu Tyr Asn Val Glu Ala Thr
    1220                1225                1230

Ser Tyr Ala Leu Leu Ala Leu Leu Gln Leu Lys Asp Phe Asp Phe
    1235                1240                1245

Val Pro Pro Val Val Arg Trp Leu Asn Glu Gln Arg Tyr Tyr Gly
    1250                1255                1260

Gly Gly Tyr Gly Ser Thr Gln Ala Thr Phe Met Val Phe Gln Ala
    1265                1270                1275

Leu Ala Gln Tyr Gln Lys Asp Ala Pro Asp His Gln Glu Leu Asn
    1280                1285                1290

Leu Asp Val Ser Leu Gln Leu Pro Ser Arg Ser Ser Lys Ile Thr
    1295                1300                1305

His Arg Ile His Trp Glu Ser Ala Ser Leu Leu Arg Ser Glu Glu
    1310                1315                1320

Thr Lys Glu Asn Glu Gly Phe Thr Val Thr Ala Glu Gly Lys Gly

1325                    1330                    1335

Gln Gly  Thr Leu Ser Val Val  Thr Met Tyr His Ala  Lys Ala Lys
    1340                    1345                    1350

Asp Gln  Leu Thr Cys Asn Lys  Phe Asp Leu Lys Val  Thr Ile Lys
    1355                    1360                    1365

Pro Ala  Pro Glu Thr Glu Lys  Arg Pro Gln Asp Ala  Lys Asn Thr
    1370                    1375                    1380

Met Ile  Leu Glu Ile Cys Thr  Arg Tyr Arg Gly Asp  Gln Asp Ala
    1385                    1390                    1395

Thr Met  Ser Ile Leu Asp Ile  Ser Met Met Thr Gly  Phe Ala Pro
    1400                    1405                    1410

Asp Thr  Asp Asp Leu Lys Gln  Leu Ala Asn Gly Val  Asp Arg Tyr
    1415                    1420                    1425

Ile Ser  Lys Tyr Glu Leu Asp  Lys Ala Phe Ser Asp  Arg Asn Thr
    1430                    1435                    1440

Leu Ile  Ile Tyr Leu Asp Lys  Val Ser His Ser Glu  Asp Asp Cys
    1445                    1450                    1455

Leu Ala  Phe Lys Val His Gln  Tyr Phe Asn Val Glu  Leu Ile Gln
    1460                    1465                    1470

Pro Gly  Ala Val Lys Val Tyr  Ala Tyr Tyr Asn Leu  Glu Glu Ser
    1475                    1480                    1485

Cys Thr  Arg Phe Tyr His Pro  Glu Lys Glu Asp Gly  Lys Leu Asn
    1490                    1495                    1500

Lys Leu  Cys Arg Asp Glu Leu  Cys Arg Cys Ala Glu  Glu Asn Cys
    1505                    1510                    1515

Phe Ile  Gln Lys Ser Asp Asp  Lys Val Thr Leu Glu  Glu Arg Leu
    1520                    1525                    1530

Asp Lys  Ala Cys Glu Pro Gly  Val Asp Tyr Val Tyr  Lys Thr Arg
    1535                    1540                    1545

Leu Val  Lys Val Gln Leu Ser  Asn Asp Phe Asp Glu  Tyr Ile Met
    1550                    1555                    1560

Ala Ile  Glu Gln Thr Ile Lys  Ser Gly Ser Asp Glu  Val Gln Val
    1565                    1570                    1575

Gly Gln  Gln Arg Thr Phe Ile  Ser Pro Ile Lys Cys  Arg Glu Ala
    1580                    1585                    1590

Leu Lys Leu Glu Glu Lys Lys His Tyr Leu Met Trp Gly Leu Ser
    1595                1600                1605

Ser Asp Phe Trp Gly Glu Lys Pro Asn Leu Ser Tyr Ile Ile Gly
    1610                1615                1620

Lys Asp Thr Trp Val Glu His Trp Pro Glu Glu Asp Glu Cys Gln
    1625                1630                1635

Asp Glu Glu Asn Gln Lys Gln Cys Gln Asp Leu Gly Ala Phe Thr
    1640                1645                1650

Glu Ser Met Val Val Phe Gly Cys Pro Asn
    1655                1660

<210>  12
<211>  1663
<212>  PRT
<213>  Homo sapiens

<400>  12

<210>  13
<211>  475
<212>  PRT
<213>  Homo sapiens

<400>  13

<210>  14
<211>  434
<212>  PRT
<213>  Homo sapiens

<400>  14

Met Ser Ile Leu Lys Ile His Ala Arg Glu Ile Phe Asp Ser Arg Gly
1               5                   10                  15

Asn Pro Thr Val Glu Val Asp Leu Phe Thr Ser Lys Gly Leu Phe Arg
            20                  25                  30

Ala Ala Val Pro Ser Gly Ala Ser Thr Gly Ile Tyr Glu Ala Leu Glu
        35                  40                  45

Leu Arg Asp Asn Asp Lys Thr Arg Tyr Met Gly Lys Gly Val Ser Lys
    50                  55                  60

Ala Val Glu His Ile Asn Lys Thr Ile Ala Pro Ala Leu Val Ser Lys
65                  70                  75                  80

Lys Leu Asn Val Thr Glu Gln Glu Lys Ile Asp Lys Leu Met Ile Glu
                85                  90                  95

Met Asp Gly Thr Glu Asn Lys Ser Lys Phe Gly Ala Asn Ala Ile Leu
            100                 105                 110

Gly Val Ser Leu Ala Val Cys Lys Ala Gly Ala Val Glu Lys Gly Val
        115                 120                 125

Pro Leu Tyr Arg His Ile Ala Asp Leu Ala Gly Asn Ser Glu Val Ile
    130                 135                 140

Leu Pro Val Pro Ala Phe Asn Val Ile Asn Gly Gly Ser His Ala Gly
145                 150                 155                 160

Asn Lys Leu Ala Met Gln Glu Phe Met Ile Leu Pro Val Gly Ala Ala
                165                 170                 175

Asn Phe Arg Glu Ala Met Arg Ile Gly Ala Glu Val Tyr His Asn Leu
            180                 185                 190

Lys Asn Val Ile Lys Glu Lys Tyr Gly Lys Asp Ala Thr Asn Val Gly
        195                 200                 205

Asp Glu Gly Gly Phe Ala Pro Asn Ile Leu Glu Asn Lys Glu Gly Leu
    210                 215                 220

Glu Leu Leu Lys Thr Ala Ile Gly Lys Ala Gly Tyr Thr Asp Lys Val
225                 230                 235                 240

Val Ile Gly Met Asp Val Ala Ala Ser Glu Phe Phe Arg Ser Gly Lys
                245                 250                 255

Tyr Asp Leu Asp Phe Lys Ser Pro Asp Asp Pro Ser Arg Tyr Ile Ser
            260                 265                 270

Pro Asp Gln Leu Ala Asp Leu Tyr Lys Ser Phe Ile Lys Asp Tyr Pro
        275                 280                 285

Val Val Ser Ile Glu Asp Pro Phe Asp Gln Asp Asp Trp Gly Ala Trp
    290                 295                 300

Gln Lys Phe Thr Ala Ser Ala Gly Ile Gln Val Val Gly Asp Asp Leu
305                 310                 315                 320

Thr Val Thr Asn Pro Lys Arg Ile Ala Lys Ala Val Asn Glu Lys Ser
                325                 330                 335

Cys Asn Cys Leu Leu Leu Lys Val Asn Gln Ile Gly Ser Val Thr Glu
            340                 345                 350

Ser Leu Gln Ala Cys Lys Leu Ala Gln Ala Asn Gly Trp Gly Val Met
        355                 360                 365

Val Ser His Arg Ser Gly Glu Thr Glu Asp Thr Phe Ile Ala Asp Leu
    370                 375                 380

Val Val Gly Leu Cys Thr Gly Gln Ile Lys Thr Gly Ala Pro Cys Arg
385                 390                 395                 400

Ser Glu Arg Leu Ala Lys Tyr Asn Gln Leu Leu Arg Ile Glu Glu Glu
                405                 410                 415

Leu Gly Ser Lys Ala Lys Phe Ala Gly Arg Asn Phe Arg Asn Pro Leu
            420                 425                 430

Ala Lys

<210>  15
<211>  434
<212>  PRT
<213>  Homo sapiens

<400>  15

Met Ser Ile Leu Lys Ile His Ala Arg Glu Ile Phe Asp Ser Arg Gly
1               5                   10                  15

Asn Pro Thr Val Glu Val Asp Leu Phe Thr Ser Lys Gly Leu Phe Arg
            20                  25                  30

Ala Ala Val Pro Ser Gly Ala Ser Thr Gly Ile Tyr Glu Ala Leu Glu
        35                  40                  45

Leu Arg Asp Asn Asp Lys Ala Arg Tyr Met Gly Lys Gly Val Ser Lys
    50                  55                  60

Ala Val Glu His Ile Asn Lys Thr Ile Ala Pro Ala Leu Val Ser Lys
65                  70                  75                  80

Lys Leu Asn Val Thr Glu Gln Glu Lys Ile Asp Lys Leu Met Ile Glu
                85                  90                  95

Met Asp Gly Thr Glu Asn Lys Ser Lys Phe Gly Ala Asn Ala Ile Leu
            100                 105                 110

Gly Val Ser Leu Ala Val Cys Lys Ala Gly Ala Val Glu Lys Gly Val
        115                 120                 125

Pro Leu Tyr Arg His Ile Ala Asp Leu Ala Gly Asn Ser Glu Val Ile
    130                 135                 140

Leu Pro Val Pro Ala Phe Asn Val Ile Asn Gly Gly Ser His Ala Gly
145                 150                 155                 160

Asn Lys Leu Ala Met Gln Glu Phe Met Ile Leu Pro Val Gly Ala Ala
                165                 170                 175

Asn Phe Arg Glu Ala Met Arg Ile Gly Ala Gly Val Tyr His Asn Leu
            180                 185                 190

Lys Asn Val Ile Lys Glu Lys Tyr Gly Lys Asp Ala Thr Asn Val Gly

195                 200                 205

Asp Glu Gly Gly Phe Ala Pro Asn Ile Leu Glu Asn Lys Glu Gly Leu
    210                 215                 220

Glu Leu Leu Lys Thr Ala Ile Gly Lys Ala Gly Tyr Thr Asp Lys Val
225                 230                 235                 240

Val Ile Gly Met Asp Val Ala Ala Ser Glu Phe Phe Arg Ser Gly Lys
                245                 250                 255

Tyr Asp Leu Asp Phe Lys Ser Pro Asp Asp Pro Ser Arg Tyr Ile Ser
            260                 265                 270

Pro Asp Gln Leu Ala Asp Leu Tyr Lys Ser Phe Ile Lys Asp Tyr Pro
        275                 280                 285

Val Val Ser Ile Glu Asp Pro Phe Asp Gln Asp Asp Trp Gly Ala Trp
    290                 295                 300

Gln Lys Phe Thr Ala Ile Ala Gly Ile Gln Val Val Gly Asp Asp Leu
305                 310                 315                 320

Thr Val Thr Asn Pro Lys Arg Ile Ala Lys Ala Val Asn Glu Lys Ser
                325                 330                 335

Cys Asn Cys Leu Leu Leu Lys Val Asn Gln Ile Gly Ser Val Thr Glu
            340                 345                 350

Ser Leu Gln Ala Cys Lys Leu Ala Gln Ala Asn Gly Trp Gly Val Met
        355                 360                 365

Val Ser His Arg Ser Gly Glu Thr Glu Asp Thr Phe Ile Ala Asp Leu
    370                 375                 380

Val Val Gly Leu Cys Thr Gly Gln Ile Lys Thr Gly Ala Pro Cys Arg
385                 390                 395                 400

Ser Glu Arg Leu Ala Lys Tyr Asn Gln Leu Leu Arg Ile Glu Glu Glu
                405                 410                 415

Leu Gly Ser Lys Ala Lys Phe Ala Gly Arg Asn Phe Arg Asn Pro Leu
            420                 425                 430

Ala Lys

<210>  16
<211>  435
<212>  PRT
<213>  Artificial

<220>

<223>  Synthetic construct

<400>  16

Met Ser Ile Leu Lys Ile His Ala Arg Glu Ile Phe Asp Ser Arg Gly
1               5                   10                  15

Asn Pro Thr Val Glu Val Asp Leu Phe Thr Ser Lys Gly Leu Phe Arg
            20                  25                  30

Ala Ala Val Pro Ser Gly Ala Ser Thr Gly Ile Tyr Glu Ala Leu Glu
        35                  40                  45

Leu Arg Asp Asn Asp Lys Thr Arg Tyr Met Gly Lys Gly Val Ser Lys
    50                  55                  60

Ala Val Glu His Ile Asn Lys Thr Ile Ala Pro Ala Leu Val Ser Lys
65                  70                  75                  80

Lys Leu Asn Val Thr Glu Gln Glu Lys Ile Asp Lys Leu Met Ile Glu
                85                  90                  95

Met Asp Gly Thr Glu Asn Lys Ser Lys Phe Gly Ala Asn Ala Ile Leu
            100                 105                 110

Gly Val Ser Leu Ala Val Cys Lys Ala Gly Ala Val Glu Lys Gly Val
        115                 120                 125

Pro Leu Tyr Arg His Ile Ala Asp Leu Ala Gly Asn Ser Glu Val Ile
    130                 135                 140

Leu Pro Val Pro Ala Phe Asn Val Ile Asn Gly Gly Ser His Ala Gly
145                 150                 155                 160

Asn Lys Leu Ala Met Gln Glu Phe Met Ile Leu Pro Val Gly Ala Ala
                165                 170                 175

Asn Phe Arg Glu Ala Met Arg Ile Gly Ala Glu Val Tyr His Asn Leu
            180                 185                 190

Lys Asn Val Ile Lys Glu Lys Tyr Gly Lys Asp Ala Thr Asn Val Gly
        195                 200                 205

Asp Glu Gly Gly Phe Ala Pro Asn Ile Leu Glu Asn Lys Glu Gly Leu
    210                 215                 220

Glu Leu Leu Lys Thr Ala Ile Gly Lys Ala Gly Tyr Thr Asp Lys Val
225                 230                 235                 240

Val Ile Gly Met Asp Val Ala Ala Ser Glu Phe Phe Arg Ser Gly Lys
                245                 250                 255

Tyr Asp Leu Asp Phe Lys Ser Pro Asp Asp Pro Ser Arg Tyr Ile Ser

260                 265                 270

Pro Asp Gln Leu Ala Asp Leu Tyr Lys Ser Phe Ile Lys Asp Tyr Pro
        275                 280                 285

Val Val Ser Ile Glu Asp Pro Phe Asp Gln Asp Asp Trp Gly Ala Trp
    290                 295                 300

Gln Lys Phe Thr Ala Ser Ala Gly Ile Gln Val Val Gly Asp Asp Leu
305                 310                 315                 320

Thr Val Thr Asn Pro Lys Arg Ile Ala Lys Ala Val Asn Glu Lys Ser
                325                 330                 335

Cys Asn Cys Leu Leu Leu Lys Val Asn Gln Ile Gly Ser Val Thr Glu
            340                 345                 350

Ser Leu Gln Ala Cys Lys Leu Ala Gln Ala Asn Gly Trp Gly Val Met
        355                 360                 365

Val Ser His Arg Ser Gly Glu Thr Glu Asp Thr Phe Ile Ala Asp Leu
    370                 375                 380

Val Val Gly Leu Cys Thr Gly Gln Ile Lys Thr Gly Ala Pro Cys Arg
385                 390                 395                 400

Ser Glu Arg Leu Ala Lys Tyr Asn Gln Leu Leu Arg Ile Glu Glu Glu
                405                 410                 415

Leu Gly Ser Lys Ala Lys Phe Ala Gly Arg Asn Phe Arg Asn Pro Leu
            420                 425                 430

Ala Lys Leu
        435

<210>  17
<211>  143
<212>  PRT
<213>  Homo sapiens

<400>  17

Ser Leu Gly Phe Tyr Phe Asp Arg Asp Asp Val Ala Leu Glu Gly Val
1               5                   10                  15

Ser His Phe Phe Arg Glu Leu Ala Glu Glu Lys Arg Glu Gly Tyr Glu
            20                  25                  30

Arg Leu Leu Lys Met Gln Asn Gln Arg Gly Gly Arg Ala Leu Phe Gln
        35                  40                  45

Asp Ile Lys Lys Pro Ala Glu Asp Glu Trp Gly Lys Thr Pro Asp Ala
    50                  55                  60

Met Lys Ala Ala Met Ala Leu Glu Lys Lys Leu Asn Gln Ala Leu Leu
65                  70                  75                  80

Asp Leu His Ala Leu Gly Ser Ala Arg Thr Asp Pro His Leu Cys Asp
                85                  90                  95

Phe Leu Glu Thr His Phe Leu Asp Glu Glu Val Lys Leu Ile Lys Lys
            100                 105                 110

Met Gly Asp His Leu Thr Asn Leu His Arg Leu Gly Gly Pro Glu Ala
        115                 120                 125

Gly Leu Gly Glu Tyr Leu Phe Glu Arg Leu Thr Leu Lys His Asp
    130                 135                 140

<210>  18
<211>  445
<212>  PRT
<213>  Homo sapiens

<400>  18

Met Asn Glu Glu Tyr Asp Val Ile Val Leu Gly Thr Gly Leu Thr Glu
1               5                   10                  15

Cys Ile Leu Ser Gly Ile Met Ser Val Asn Gly Lys Lys Val Leu His
            20                  25                  30

Met Asp Arg Asn Pro Tyr Tyr Gly Gly Glu Ser Ala Ser Ile Thr Pro
        35                  40                  45

Leu Glu Asp Leu Tyr Lys Arg Phe Lys Ile Pro Gly Ser Pro Pro Glu
    50                  55                  60

Ser Met Gly Arg Gly Arg Asp Trp Asn Val Asp Leu Ile Pro Lys Phe
65                  70                  75                  80

Leu Met Ala Asn Gly Gln Leu Val Lys Met Leu Leu Tyr Thr Glu Val
                85                  90                  95

Thr Arg Tyr Leu Asp Phe Lys Val Thr Glu Gly Ser Phe Val Tyr Lys
            100                 105                 110

Gly Gly Lys Ile Tyr Lys Val Pro Ser Thr Glu Ala Glu Ala Leu Ala
        115                 120                 125

Ser Ser Leu Met Gly Leu Phe Glu Lys Arg Arg Phe Arg Lys Phe Leu
    130                 135                 140

Val Tyr Val Ala Asn Phe Asp Glu Lys Asp Pro Arg Thr Phe Glu Gly
145                 150                 155                 160

Ile Asp Pro Lys Lys Thr Thr Met Arg Asp Val Tyr Lys Lys Phe Asp

                165                 170                 175

Leu Gly Gln Asp Val Ile Asp Phe Thr Gly His Ala Leu Ala Leu Tyr
            180                 185                 190

Arg Thr Asp Asp Tyr Leu Asp Gln Pro Cys Tyr Glu Thr Ile Asn Arg
        195                 200                 205

Ile Lys Leu Tyr Ser Glu Ser Leu Ala Arg Tyr Gly Lys Ser Pro Tyr
    210                 215                 220

Leu Tyr Pro Leu Tyr Gly Leu Gly Glu Leu Pro Gln Gly Phe Ala Arg
225                 230                 235                 240

Leu Ser Ala Ile Tyr Gly Gly Thr Tyr Met Leu Asn Lys Pro Ile Glu
                245                 250                 255

Glu Ile Ile Val Gln Asn Gly Lys Val Ile Gly Val Lys Ser Glu Gly
            260                 265                 270

Glu Ile Ala Arg Cys Lys Gln Leu Ile Cys Asp Pro Ser Tyr Val Lys
        275                 280                 285

Asp Arg Val Glu Lys Val Gly Gln Val Ile Arg Val Ile Cys Ile Leu
    290                 295                 300

Ser His Pro Ile Lys Asn Thr Asn Asp Ala Asn Ser Cys Gln Ile Ile
305                 310                 315                 320

Ile Pro Gln Asn Gln Val Asn Arg Lys Ser Asp Ile Tyr Val Cys Met
                325                 330                 335

Ile Ser Phe Ala His Asn Val Ala Ala Gln Gly Lys Tyr Ile Ala Ile
            340                 345                 350

Val Ser Thr Thr Val Glu Thr Lys Glu Pro Glu Lys Glu Ile Arg Pro
        355                 360                 365

Ala Leu Glu Leu Leu Glu Pro Ile Glu Gln Lys Phe Val Ser Ile Ser
    370                 375                 380

Asp Leu Leu Val Pro Lys Asp Leu Gly Thr Glu Ser Gln Ile Phe Ile
385                 390                 395                 400

Ser Arg Thr Tyr Asp Ala Thr Thr His Phe Glu Thr Thr Cys Asp Asp
                405                 410                 415

Ile Lys Asn Ile Tyr Lys Arg Met Thr Gly Ser Glu Phe Asp Phe Glu
            420                 425                 430

Glu Met Lys Arg Lys Lys Asn Asp Ile Tyr Gly Glu Asp
        435                 440                 445

<210>  19
<211>  145
<212>  PRT
<213>  Homo sapiens

<400>  19

Leu Asn Ala Leu Gln Glu Glu Leu Ala Pro Phe Gly Leu Val Ile Leu
1               5                   10                  15

Gly Phe Pro Cys Asn Gln Phe Gly Lys Gln Glu Pro Gly Glu Asn Ser
            20                  25                  30

Glu Ile Leu Pro Thr Leu Lys Tyr Val Arg Pro Gly Gly Gly Phe Val
        35                  40                  45

Pro Asn Phe Gln Leu Phe Glu Lys Gly Asp Val Asn Gly Glu Lys Glu
    50                  55                  60

Gln Lys Phe Tyr Thr Phe Leu Lys Asn Ser Cys Pro Pro Thr Ser Glu
65                  70                  75                  80

Leu Leu Gly Thr Ser Asp Arg Leu Phe Trp Glu Pro Met Lys Val His
                85                  90                  95

Asp Ile Arg Trp Asn Phe Glu Lys Phe Leu Val Gly Pro Asp Gly Ile
            100                 105                 110

Pro Ile Met Arg Trp His His Arg Thr Thr Val Ser Asn Val Lys Met
        115                 120                 125

Asp Ile Leu Ser Tyr Met Arg Arg Gln Ala Ala Leu Gly Val Lys Arg
    130                 135                 140

Lys
145

<210>  20
<211>  474
<212>  PRT
<213>  Homo sapiens

<400>  20

<210>  21
<211>  208
<212>  PRT
<213>  Homo sapiens

<400>  21

Pro Pro Tyr Thr Val Val Tyr Phe Pro Val Arg Gly Arg Cys Ala Ala
1               5                   10                  15

Leu Arg Met Leu Leu Ala Asp Gln Gly Gln Ser Trp Lys Glu Glu Val
            20                  25                  30

Val Thr Val Glu Thr Trp Gln Glu Gly Ser Leu Lys Ala Ser Cys Leu
        35                  40                  45

Tyr Gly Gln Leu Pro Lys Phe Gln Asp Gly Asp Leu Thr Leu Tyr Gln
    50                  55                  60

Ser Asn Thr Ile Leu Arg His Leu Gly Arg Thr Leu Gly Leu Tyr Gly
65                  70                  75                  80

Lys Asp Gln Gln Glu Ala Ala Leu Val Asp Met Val Asn Asp Gly Val
                85                  90                  95

Glu Asp Leu Arg Cys Lys Tyr Ile Ser Leu Ile Tyr Thr Asn Tyr Glu
            100                 105                 110

Ala Gly Lys Asp Asp Tyr Val Lys Ala Leu Pro Gly Gln Leu Lys Pro
        115                 120                 125

Phe Glu Thr Leu Leu Ser Gln Asn Gln Gly Gly Lys Thr Phe Ile Val
    130                 135                 140

Gly Asp Gln Ile Ser Phe Ala Asp Tyr Asn Leu Leu Asp Leu Leu Leu
145                 150                 155                 160

Ile His Glu Val Leu Ala Pro Gly Cys Leu Asp Ala Phe Pro Leu Leu
                165                 170                 175

Ser Ala Tyr Val Gly Arg Leu Ser Ala Arg Pro Lys Leu Lys Ala Phe
            180                 185                 190

Leu Ala Ser Pro Glu Tyr Val Asn Leu Pro Met Asp Glu Lys Ser Leu
        195                 200                 205

<210>  22
<211>  414
<212>  PRT
<213>  Homo sapiens

<400>  22

<210>  23
<211>  348
<212>  PRT
<213>  Homo sapiens

<400>  23

Tyr Tyr Tyr Gly Met Asp Val Trp Gly Gln Gly Thr Thr Val Thr Val
1               5                   10                  15

Ser Ser Ala Ser Thr Lys Gly Pro Ser Val Phe Pro Leu Ala Pro Ser
            20                  25                  30

Ser Lys Ser Thr Ser Gly Gly Thr Ala Ala Leu Gly Cys Leu Val Lys
        35                  40                  45

Asp Tyr Phe Pro Glu Pro Val Thr Val Ser Trp Asn Ser Gly Ala Leu
    50                  55                  60

Thr Ser Gly Val His Thr Phe Pro Ala Val Leu Gln Ser Ser Gly Leu
65                  70                  75                  80

Tyr Ser Leu Ser Ser Val Val Thr Val Pro Ser Ser Ser Leu Gly Thr
                85                  90                  95

Gln Thr Tyr Ile Cys Asn Val Asn His Lys Pro Ser Asn Thr Lys Val
            100                 105                 110

Asp Lys Arg Val Glu Pro Lys Ser Cys Asp Lys Thr His Thr Cys Pro
        115                 120                 125

Pro Cys Pro Ala Pro Glu Leu Leu Gly Gly Pro Ser Val Phe Leu Phe
    130                 135                 140

Pro Pro Lys Pro Lys Asp Thr Leu Met Ile Ser Arg Thr Pro Glu Val
145                 150                 155                 160

Thr Cys Val Val Val Asp Val Ser His Glu Asp Pro Glu Val Lys Phe
                165                 170                 175

Asn Trp Tyr Val Asp Gly Val Glu Val His Asn Ala Lys Thr Lys Pro
            180                 185                 190

Arg Glu Glu Gln Tyr Asn Ser Thr Tyr Arg Val Val Ser Val Leu Thr
        195                 200                 205

Val Leu His Gln Asp Trp Leu Asn Gly Lys Glu Tyr Lys Cys Lys Val
    210                 215                 220

Ser Asn Lys Ala Leu Pro Ala Pro Ile Glu Lys Thr Ile Ser Lys Ala
225                 230                 235                 240

Lys Gly Gln Pro Arg Glu Pro Gln Val Tyr Thr Leu Pro Pro Ser Arg

245                 250                 255

Glu Glu Met Thr Lys Asn Gln Val Ser Leu Thr Cys Leu Val Lys Gly
            260                 265                 270

Phe Tyr Pro Ser Asp Ile Ala Val Glu Trp Glu Ser Asn Gly Gln Pro
        275                 280                 285

Glu Asn Asn Tyr Lys Thr Thr Pro Pro Val Leu Asp Ser Asp Gly Ser
    290                 295                 300

Phe Phe Leu Tyr Ser Lys Leu Thr Val Asp Lys Ser Arg Trp Gln Gln
305                 310                 315                 320

Gly Asn Val Phe Ser Cys Ser Val Met His Glu Ala Leu His Asn His
                325                 330                 335

Tyr Thr Gln Lys Ser Leu Ser Leu Ser Pro Gly Lys
            340                 345

<210>  24
<211>  356
<212>  PRT
<213>  Homo sapiens

<400>  24

Met Ala Ala Glu Gly Trp Ile Trp Arg Trp Gly Trp Gly Arg Arg Cys
1               5                   10                  15

Leu Gly Arg Pro Gly Leu Leu Gly Pro Gly Pro Gly Pro Thr Thr Pro
            20                  25                  30

Leu Phe Leu Leu Leu Leu Leu Gly Ser Val Thr Ala Asp Ile Thr Asp
        35                  40                  45

Gly Asn Ser Glu His Leu Lys Arg Glu His Ser Leu Ile Lys Pro Tyr
    50                  55                  60

Gln Gly Val Gly Ser Ser Ser Met Pro Leu Trp Asp Phe Gln Gly Ser
65                  70                  75                  80

Thr Met Leu Thr Ser Gln Tyr Val Arg Leu Thr Pro Asp Glu Arg Ser
                85                  90                  95

Lys Glu Gly Ser Ile Trp Asn His Gln Pro Cys Phe Leu Lys Asp Trp
            100                 105                 110

Glu Met His Val His Phe Lys Val His Gly Thr Gly Lys Lys Asn Leu
        115                 120                 125

His Gly Asp Gly Ile Ala Leu Trp Tyr Thr Arg Asp Arg Leu Val Pro
    130                 135                 140

Gly Pro Val Phe Gly Ser Lys Asp Asn Phe His Gly Leu Ala Ile Phe
145                 150                 155                 160

Leu Asp Thr Tyr Pro Asn Asp Glu Thr Thr Glu Arg Val Phe Pro Tyr
                165                 170                 175

Ile Ser Val Met Val Asn Asn Gly Ser Leu Ser Tyr Asp His Ser Lys
            180                 185                 190

Asp Gly Arg Trp Thr Glu Leu Ala Gly Cys Thr Ala Asp Phe Arg Asn
        195                 200                 205

Arg Asp His Asp Thr Phe Leu Ala Val Arg Tyr Ser Arg Gly Arg Leu
    210                 215                 220

Thr Val Met Thr Asp Leu Glu Asp Lys Asn Glu Trp Lys Asn Cys Ile
225                 230                 235                 240

Asp Ile Thr Gly Val Arg Leu Pro Thr Gly Tyr Tyr Phe Gly Ala Ser
                245                 250                 255

Ala Gly Thr Gly Asp Leu Ser Asp Asn His Asp Ile Ile Ser Met Lys
            260                 265                 270

Leu Phe Gln Leu Met Val Glu His Thr Pro Asp Glu Glu Ser Ile Asp
        275                 280                 285

Trp Thr Lys Ile Glu Pro Ser Val Asn Phe Leu Lys Ser Pro Lys Asp
    290                 295                 300

Asn Val Asp Asp Pro Thr Gly Asn Phe Arg Ser Gly Pro Leu Thr Gly
305                 310                 315                 320

Trp Arg Val Phe Leu Leu Leu Leu Cys Ala Leu Leu Gly Ile Val Val
                325                 330                 335

Cys Ala Val Val Gly Ala Val Val Phe Gln Lys Arg Gln Glu Arg Asn
            340                 345                 350

Lys Arg Phe Tyr
        355

<210>  25
<211>  128
<212>  PRT
<213>  Homo sapiens

<400>  25

Met Gly Thr Ser Ser Ile Phe Leu Cys Val Leu Phe Leu Cys Gly Ala
1               5                   10                  15

Leu Gly Leu Thr Met Ser Pro Ala Arg Gly Arg Leu Arg Cys Tyr Ile

            20                  25                  30

Cys Gly Phe Thr Lys Pro Cys His Pro Val Pro Thr Glu Cys Arg Asp
        35                  40                  45

Asp Glu Ala Cys Gly Ile Ser Ile Gly Thr Ser Val Lys Lys Leu Pro
    50                  55                  60

Leu Lys Gly Pro Val Pro Ser Ala Arg Leu Cys His Leu Leu Ala Ala
65                  70                  75                  80

Leu Leu His Ser Val Ala Pro Leu Leu Arg Ala Gly Pro Val Gln His
                85                  90                  95

Ser Arg Phe Pro Thr Ala Ala His Gln Pro Pro Arg Leu Pro Ala Pro
            100                 105                 110

Leu Cys Gly Gln Leu Arg Trp Glu Arg Thr Pro Pro Pro Leu Ala Ser
        115                 120                 125

<210> 26
<211> 170
<212> PRT
<213> Homo sapiens

<400> 26

Thr Pro Leu Gly Pro Lys Trp Pro Glu Pro Val Phe Gly Arg Leu Ala
1               5                   10                  15

Ser Pro Gly Phe Pro Gly Glu Tyr Ala Asn Asp Gln Glu Arg Arg Trp
            20                  25                  30

Thr Leu Thr Ala Pro Pro Gly Tyr Arg Leu Arg Leu Tyr Phe Thr His
        35                  40                  45

Phe Asp Leu Glu Leu Ser His Leu Cys Glu Tyr Asp Phe Val Lys Leu
    50                  55                  60

Ser Ser Gly Ala Lys Val Leu Ala Thr Leu Cys Gly Gln Glu Ser Thr
65                  70                  75                  80

Asp Thr Glu Arg Ala Pro Gly Lys Asp Thr Phe Tyr Ser Leu Gly Ser
                85                  90                  95

Ser Leu Asp Ile Thr Phe Arg Ser Asp Tyr Ser Asn Glu Lys Pro Phe
            100                 105                 110

Thr Gly Phe Glu Ala Phe Tyr Ala Ala Glu Asp Ile Asp Glu Cys Gln
        115                 120                 125

Val Ala Pro Gly Glu Ala Pro Thr Cys Asp His His Cys His Asn His
    130                 135                 140

Leu Gly Gly Phe Tyr Cys Ser Cys Arg Ala Gly Tyr Val Leu His Arg
145                 150                 155                 160

Asn Lys Arg Thr Cys Ser Glu Gln Ser Leu
                165                 170

<210>  27
<211>  297
<212>  PRT
<213>  Homo sapiens

<400>  27

Met Asp Ala Glu Gly Leu Ala Leu Leu Leu Pro Pro Val Thr Leu Ala
1               5                   10                  15

Ala Leu Val Asp Ser Trp Leu Arg Glu Asp Cys Pro Gly Leu Asn Tyr
            20                  25                  30

Ala Ala Leu Val Ser Gly Ala Gly Pro Ser Gln Ala Ala Leu Trp Ala
        35                  40                  45

Lys Ser Pro Gly Val Leu Ala Gly Gln Pro Phe Phe Asp Ala Ile Phe
    50                  55                  60

Thr Gln Leu Asn Cys Gln Val Ser Trp Phe Leu Pro Glu Gly Ser Lys
65                  70                  75                  80

Leu Val Pro Val Ala Arg Val Ala Glu Val Arg Gly Pro Ala His Cys
                85                  90                  95

Leu Leu Leu Gly Glu Arg Val Ala Leu Asn Thr Leu Ala Arg Cys Ser
            100                 105                 110

Gly Ile Ala Ser Ala Ala Ala Ala Ala Val Glu Ala Ala Arg Gly Ala
        115                 120                 125

Gly Trp Thr Gly His Val Ala Gly Thr Arg Lys Thr Thr Pro Gly Phe
    130                 135                 140

Arg Leu Val Glu Lys Tyr Gly Leu Leu Val Gly Gly Ala Ala Ser His
145                 150                 155                 160

Arg Tyr Asp Leu Gly Gly Leu Val Met Val Lys Asp Asn His Val Val
                165                 170                 175

Ala Ala Gly Gly Val Glu Lys Ala Val Arg Ala Ala Arg Gln Ala Ala
            180                 185                 190

Asp Phe Ala Leu Lys Val Glu Val Glu Cys Ser Ser Leu Gln Glu Ala
        195                 200                 205

Val Gln Ala Ala Glu Ala Gly Ala Asp Leu Val Leu Leu Asp Asn Phe

210                 215                 220

Lys Pro Glu Glu Leu His Pro Thr Ala Thr Val Leu Lys Ala Gln Phe
225                 230                 235                 240

Pro Ser Val Ala Val Glu Ala Ser Gly Gly Ile Thr Leu Asp Asn Leu
                245                 250                 255

Pro Gln Phe Cys Gly Pro His Ile Asp Val Ile Ser Met Gly Met Leu
            260                 265                 270

Thr Gln Ala Ala Pro Ala Leu Asp Phe Ser Leu Lys Leu Phe Ala Lys
        275                 280                 285

Glu Val Ala Pro Val Pro Lys Ile His
    290                 295

<210>  28
<211>  420
<212>  PRT
<213>  Homo sapiens

<400>  28

<210>  29
<211>  373
<212>  PRT
<213>  Homo sapiens

<400>  29

Met Ser Gly Glu Asp Glu Gln Gln Glu Gln Thr Ile Ala Glu Asp Leu
1               5                   10                  15

Val Val Thr Lys Tyr Lys Met Gly Gly Asp Ile Ala Asn Arg Val Leu
            20                  25                  30

Arg Ser Leu Val Glu Ala Ser Ser Ser Gly Val Ser Val Leu Ser Leu
        35                  40                  45

Cys Glu Lys Gly Asp Ala Met Ile Met Glu Glu Thr Gly Lys Ile Phe
    50                  55                  60

Lys Lys Glu Lys Glu Met Lys Lys Gly Ile Ala Phe Pro Thr Ser Ile
65                  70                  75                  80

Ser Val Asn Asn Cys Val Cys His Phe Ser Pro Leu Lys Ser Asp Gln
                85                  90                  95

Asp Tyr Ile Leu Lys Glu Gly Asp Leu Val Lys Ile Asp Leu Gly Val
            100                 105                 110

His Val Asp Gly Phe Ile Ala Asn Val Ala His Thr Phe Val Val Asp
        115                 120                 125

Val Ala Gln Gly Thr Gln Val Thr Gly Arg Lys Ala Asp Val Ile Lys
    130                 135                 140

Ala Ala His Leu Cys Ala Glu Ala Ala Leu Arg Leu Val Lys Pro Gly
145                 150                 155                 160

Asn Gln Asn Thr Gln Val Thr Glu Ala Trp Asn Lys Val Ala His Ser
                165                 170                 175

Phe Asn Cys Thr Pro Ile Glu Gly Met Leu Ser His Gln Leu Lys Gln
            180                 185                 190

His Val Ile Asp Gly Glu Lys Thr Ile Ile Gln Asn Pro Thr Asp Gln
        195                 200                 205

Gln Lys Lys Asp His Glu Lys Ala Glu Phe Glu Val His Glu Val Tyr
    210                 215                 220

Ala Val Asp Val Leu Val Ser Ser Gly Glu Gly Lys Ala Lys Asp Ala
225                 230                 235                 240

Gly Gln Arg Thr Thr Ile Tyr Lys Arg Asp Pro Ser Lys Gln Tyr Gly
                245                 250                 255

Leu Lys Met Lys Thr Ser Arg Ala Phe Phe Ser Glu Val Glu Arg Arg
            260                 265                 270

Phe Asp Ala Met Pro Phe Thr Leu Arg Ala Phe Glu Asp Glu Lys Lys
        275                 280                 285

Ala Arg Met Gly Val Val Glu Cys Ala Lys His Glu Leu Leu Gln Pro
    290                 295                 300

Phe Asn Val Leu Tyr Glu Lys Glu Gly Glu Phe Val Ala Gln Phe Lys
305                 310                 315                 320

Phe Thr Val Leu Leu Met Pro Asn Gly Pro Met Arg Ile Thr Ser Gly
                325                 330                 335

Pro Phe Glu Pro Asp Leu Tyr Lys Ser Glu Met Glu Val Gln Asp Ala
            340                 345                 350

Glu Leu Lys Ala Leu Leu Gln Ser Ser Ala Ser Arg Lys Thr Gln Lys
        355                 360                 365

Lys Lys Lys Lys Lys
    370

<210>  30
<211>  189
<212>  PRT
<213>  Homo sapiens

<220>
<221>  misc_feature
<222>  (106)..(106)
<223>  Xaa can be any naturally occurring amino acid

<400>  30

Met Ala Ser Lys Arg Ala Leu Val Ile Leu Ala Lys Gly Ala Glu Glu
1               5                   10                  15

Met Glu Thr Val Ile Pro Val Asp Val Met Arg Arg Ala Gly Ile Lys
            20                  25                  30

Val Thr Val Ala Gly Leu Ala Gly Lys Asp Pro Val Gln Cys Ser Arg
        35                  40                  45

Asp Val Val Ile Cys Pro Asp Ala Ser Leu Glu Asp Ala Lys Lys Glu
    50                  55                  60

Gly Pro Tyr Asp Val Val Val Leu Pro Gly Gly Asn Leu Gly Ala Gln
65                  70                  75                  80

Asn Leu Ser Glu Ser Ala Ala Val Lys Glu Ile Leu Lys Glu Gln Glu
                85                  90                  95

Asn Arg Lys Gly Leu Ile Ala Ala Ile Xaa Ala Gly Pro Thr Ala Leu
            100                 105                 110

Leu Ala His Glu Ile Gly Phe Gly Ser Lys Val Thr Thr His Pro Leu
        115                 120                 125

Ala Lys Asp Lys Met Met Asn Gly Gly His Tyr Thr Tyr Ser Glu Asn
    130                 135                 140

Arg Val Glu Lys Asp Gly Leu Ile Leu Thr Ser Arg Gly Pro Gly Thr
145                 150                 155                 160

Ser Phe Glu Phe Ala Leu Ala Ile Val Glu Ala Leu Asn Gly Lys Glu
                165                 170                 175

Val Ala Ala Gln Val Lys Ala Pro Leu Val Leu Lys Asp
            180                 185

<210> 31
<211> 356
<212> PRT
<213> Homo sapiens

<400> 31

Met Asp Ala Gly Val Thr Glu Ser Gly Leu Asn Val Thr Leu Thr Ile
1               5                   10                  15

Arg Leu Leu Met His Gly Lys Glu Val Gly Ser Ile Ile Gly Lys Lys
            20                  25                  30

Gly Glu Ser Val Lys Arg Ile Arg Glu Glu Ser Gly Ala Arg Ile Asn
        35                  40                  45

Ile Ser Glu Gly Asn Cys Pro Glu Arg Ile Ile Thr Leu Thr Gly Pro
    50                  55                  60

Thr Asn Ala Ile Phe Lys Ala Phe Ala Met Ile Ile Asp Lys Leu Glu
65                  70                  75                  80

Glu Asp Ile Asn Ser Ser Met Thr Asn Ser Thr Ala Ala Ser Arg Pro
                85                  90                  95

Pro Val Thr Leu Arg Leu Val Val Pro Ala Thr Gln Cys Gly Ser Leu
            100                 105                 110

Ile Gly Lys Gly Gly Cys Lys Ile Lys Glu Ile Arg Glu Ser Thr Gly
        115                 120                 125

Ala Gln Val Gln Val Ala Gly Asp Met Leu Pro Asn Ser Thr Glu Arg
    130                 135                 140

Ala Ile Thr Ile Ala Gly Val Pro Gln Ser Val Thr Glu Cys Val Lys
145                 150                 155                 160

Gln Ile Cys Leu Val Met Leu Glu Thr Leu Ser Gln Ser Pro Gln Gly
                165                 170                 175

Arg Val Met Thr Ile Pro Tyr Gln Pro Met Pro Ala Ser Ser Pro Val
            180                 185                 190

Ile Cys Ala Gly Gly Gln Asp Arg Cys Ser Asp Ala Val Gly Tyr Pro
        195                 200                 205

His Ala Thr His Asp Leu Glu Gly Pro Pro Leu Asp Ala Tyr Ser Ile
    210                 215                 220

Gln Gly Gln His Thr Ile Ser Pro Leu Asp Leu Ala Lys Leu Asn Gln
225                 230                 235                 240

Val Ala Arg Gln Gln Ser His Phe Ala Met Met His Gly Gly Thr Gly
                245                 250                 255

Phe Ala Gly Ile Asp Ser Ser Ser Pro Glu Val Lys Gly Tyr Trp Ala
            260                 265                 270

Ser Leu Asp Ala Ser Thr Gln Thr Thr His Glu Leu Thr Ile Pro Asn
        275                 280                 285

Asn Leu Ile Gly Cys Ile Ile Gly Arg Gln Gly Ala Asn Ile Asn Glu
    290                 295                 300

Ile Arg Gln Met Ser Gly Ala Gln Ile Lys Ile Ala Asn Pro Val Glu
305                 310                 315                 320

Gly Ser Ser Gly Arg Gln Val Thr Ile Thr Gly Ser Ala Ala Ser Ile
                325                 330                 335

Ser Leu Ala Gln Tyr Leu Ile Asn Ala Arg Leu Ser Ser Glu Lys Gly
            340                 345                 350

Met Gly Cys Ser
        355

<210>  32
<211>  873
<212>  PRT
<213>  Homo sapiens

<400>  32

Met Ala Thr Phe Ile Ser Met Gln Leu Lys Lys Thr Ser Glu Val Asp
1               5                   10                  15

Leu Ala Lys Pro Leu Val Lys Phe Ile Gln Gln Thr Tyr Pro Ser Gly
            20                  25                  30

Gly Glu Glu Gln Ala Gln Tyr Cys Arg Ala Ala Glu Glu Leu Ser Lys
        35                  40                  45

Leu Arg Arg Ala Ala Val Gly Arg Pro Leu Asp Lys His Glu Gly Ala
    50                  55                  60

Leu Glu Thr Leu Leu Arg Tyr Tyr Asp Gln Ile Cys Ser Ile Glu Pro
65                  70                  75                  80

Lys Phe Pro Phe Ser Glu Asn Gln Ile Cys Leu Thr Phe Thr Trp Lys
                85                  90                  95

Asp Ala Phe Asp Lys Gly Ser Leu Phe Gly Gly Ser Val Lys Leu Ala
            100                 105                 110

Leu Ala Ser Leu Gly Tyr Glu Lys Ser Cys Val Leu Phe Asn Cys Ala
        115                 120                 125

Ala Leu Ala Ser Gln Ile Ala Ala Glu Gln Asn Leu Asp Asn Asp Glu
    130                 135                 140

Gly Leu Lys Ile Ala Ala Lys His Tyr Gln Phe Ala Ser Gly Ala Phe
145                 150                 155                 160

Leu His Ile Lys Glu Thr Val Leu Ser Ala Leu Ser Arg Glu Pro Thr
                165                 170                 175

Val Asp Ile Ser Pro Asp Thr Val Gly Thr Leu Ser Leu Ile Met Leu
            180                 185                 190

Ala Gln Ala Gln Glu Val Phe Phe Leu Lys Ala Thr Arg Asp Lys Met
        195                 200                 205

Lys Asp Ala Ile Ile Ala Lys Leu Ala Asn Gln Ala Ala Asp Tyr Phe
    210                 215                 220

Gly Asp Ala Phe Lys Gln Cys Gln Tyr Lys Asp Thr Leu Pro Lys Tyr
225                 230                 235                 240

Phe Tyr Phe Gln Glu Val Phe Pro Val Leu Ala Ala Lys His Cys Ile
                245                 250                 255

Met Gln Ala Asn Ala Glu Tyr His Gln Ser Ile Leu Ala Lys Gln Gln
            260                 265                 270

Lys Lys Phe Gly Glu Glu Ile Ala Arg Leu Gln His Ala Ala Glu Leu
        275                 280                 285

Ile Lys Thr Val Ala Ser Arg Tyr Asp Glu Tyr Val Asn Val Lys Asp
    290                 295                 300

Phe Ser Asp Lys Ile Asn Arg Ala Leu Ala Ala Ala Lys Lys Asp Asn
305                 310                 315                 320

Asp Phe Ile Tyr His Asp Arg Val Pro Asp Leu Lys Asp Leu Asp Pro
                325                 330                 335

Ile Gly Lys Ala Thr Leu Val Lys Ser Thr Pro Val Asn Val Pro Ile
            340                 345                 350

Ser Gln Lys Phe Thr Asp Leu Phe Glu Lys Met Val Pro Val Ser Val
        355                 360                 365

Gln Gln Ser Leu Ala Ala Tyr Asn Gln Arg Lys Ala Asp Leu Ile Asn
    370                 375                 380

Arg Ser Ile Ala Gln Met Arg Glu Ala Thr Thr Leu Ala Asn Gly Val
385                 390                 395                 400

Leu Ala Ser Leu Asn Leu Pro Ala Ala Ile Glu Asp Val Ser Gly Asp
                405                 410                 415

Thr Val Pro Gln Ser Ile Leu Thr Lys Ser Arg Ser Val Ile Glu Gln
            420                 425                 430

Gly Gly Ile Gln Thr Val Asp Gln Leu Ile Lys Glu Leu Pro Glu Leu
        435                 440                 445

Leu Gln Arg Asn Arg Glu Ile Leu Asp Glu Ser Leu Arg Leu Leu Asp
    450                 455                 460

Glu Glu Glu Ala Thr Asp Asn Asp Leu Arg Ala Lys Phe Lys Glu Arg
465                 470                 475                 480

Trp Gln Arg Thr Pro Ser Asn Glu Leu Tyr Lys Pro Leu Arg Ala Glu
                485                 490                 495

Gly Thr Asn Phe Arg Thr Val Leu Asp Lys Ala Val Gln Ala Asp Gly
            500                 505                 510

Gln Val Lys Glu Cys Tyr Gln Ser His Arg Asp Thr Ile Val Leu Leu
        515                 520                 525

Cys Lys Pro Glu Pro Glu Leu Asn Ala Ala Ile Pro Ser Ala Asn Pro
    530                 535                 540

Ala Lys Thr Met Gln Gly Ser Glu Val Val Asn Val Leu Lys Ser Leu
545                 550                 555                 560

Leu Ser Asn Leu Asp Glu Val Lys Lys Glu Arg Glu Gly Leu Glu Asn
                565                 570                 575

Asp Leu Lys Ser Val Asn Phe Asp Met Thr Ser Lys Phe Leu Thr Ala
            580                 585                 590

Leu Ala Gln Asp Gly Val Ile Asn Glu Glu Ala Leu Ser Val Thr Glu
        595                 600                 605

Leu Asp Arg Val Tyr Gly Gly Leu Thr Thr Lys Val Gln Glu Ser Leu
    610                 615                 620

Lys Lys Gln Glu Gly Leu Leu Lys Asn Ile Gln Val Ser His Gln Glu
625                 630                 635                 640

Phe Ser Lys Met Lys Gln Ser Asn Asn Glu Ala Asn Leu Arg Glu Glu
                645                 650                 655

Val Leu Lys Asn Leu Ala Thr Ala Tyr Asp Asn Phe Val Glu Leu Val
            660                 665                 670

Ala Asn Leu Lys Glu Gly Thr Lys Phe Tyr Asn Glu Leu Thr Glu Ile
        675                 680                 685

Leu Val Arg Phe Gln Asn Lys Cys Ser Asp Ile Val Phe Ala Arg Lys
    690                 695                 700

Thr Glu Arg Asp Glu Leu Leu Lys Asp Leu Gln Gln Ser Ile Ala Arg
705                 710                 715                 720

Glu Pro Ser Ala Pro Ser Ile Pro Thr Pro Ala Tyr Gln Ser Ser Pro
                725                 730                 735

Ala Gly Gly His Ala Pro Thr Pro Pro Thr Pro Ala Pro Arg Thr Met
            740                 745                 750

Pro Pro Thr Lys Pro Gln Pro Pro Ala Arg Pro Pro Pro Pro Val Leu
        755                 760                 765

Pro Ala Asn Arg Ala Pro Ser Ala Thr Ala Pro Ser Pro Val Gly Ala
    770                 775                 780

Gly Thr Ala Ala Pro Ala Pro Ser Gln Thr Pro Gly Ser Ala Pro Pro
785                 790                 795                 800

Pro Gln Ala Gln Gly Pro Pro Tyr Pro Thr Tyr Pro Gly Tyr Pro Gly
                805                 810                 815

Tyr Cys Gln Met Pro Met Pro Met Gly Tyr Asn Pro Tyr Ala Tyr Gly
            820                 825                 830

Gln Tyr Asn Met Pro Tyr Pro Pro Val Tyr His Gln Ser Pro Gly Gln
        835                 840                 845

Ala Pro Tyr Pro Gly Pro Gln Gln Pro Ser Tyr Pro Phe Pro Gln Pro

850                 855                 860

Pro Gln Gln Ser Tyr Tyr Pro Gln Gln
865                 870

<210>  33
<211>  423
<212>  PRT
<213>  Homo sapiens

<400>  33

<210>  34
<211>  164
<212>  PRT
<213>  Homo sapiens

<400>  34

Gly Lys Tyr Val Val Leu Phe Phe Tyr Pro Leu Asp Phe Thr Phe Val
1               5                   10                  15

Cys Pro Thr Glu Ile Ile Ala Phe Ser Asn Arg Ala Glu Asp Phe Arg
            20                  25                  30

Lys Leu Gly Cys Glu Val Leu Gly Val Ser Val Asp Ser Gln Phe Thr

35                  40                  45

His Leu Ala Trp Ile Asn Thr Pro Arg Lys Glu Gly Gly Leu Gly Pro
    50                  55                  60

Leu Asn Ile Pro Leu Leu Ala Asp Val Thr Arg Arg Leu Ser Glu Asp
65                  70                  75                  80

Tyr Gly Val Leu Lys Thr Asp Glu Gly Ile Ala Tyr Arg Gly Leu Phe
                85                  90                  95

Ile Ile Asp Gly Lys Gly Val Leu Arg Gln Ile Thr Val Asn Asp Leu
            100                 105                 110

Pro Val Gly Arg Ser Val Asp Glu Ala Leu Arg Leu Val Gln Ala Phe
        115                 120                 125

Gln Tyr Thr Asp Glu His Gly Glu Val Cys Pro Ala Gly Trp Lys Pro
    130                 135                 140

Gly Ser Asp Thr Ile Lys Pro Asn Val Asp Asp Ser Lys Glu Tyr Phe
145                 150                 155                 160

Ser Lys His Asn

<210>  35
<211>  310
<212>  PRT
<213>  Homo sapiens

<400>  35

Arg Ser Gly Leu Trp Val Phe Cys Tyr Arg Asp Arg Leu Val Met Gln
1               5                   10                  15

Lys Gly Leu Lys Asp Asn Phe Ala Asp Val Gln Val Ser Val Val Asp
            20                  25                  30

Cys Pro Asp Leu Thr Lys Glu Pro Phe Thr Phe Pro Val Lys Gly Ile
        35                  40                  45

Cys Gly Lys Thr Arg Ile Ala Glu Val Gly Gly Val Pro Tyr Leu Leu
    50                  55                  60

Pro Leu Val Asn Gln Lys Lys Val Tyr Asp Leu Asn Lys Ile Ala Lys
65                  70                  75                  80

Glu Ile Lys Leu Pro Gly Ala Phe Ile Leu Gly Ala Gly Ala Gly Pro
                85                  90                  95

Phe Gln Thr Leu Gly Phe Asn Ser Glu Phe Met Pro Val Ile Gln Thr
            100                 105                 110

Glu Ser Glu His Lys Pro Pro Val Asn Gly Ser Tyr Phe Ala His Val
        115                 120                 125

Asn Pro Ala Asp Gly Gly Cys Leu Leu Glu Lys Tyr Ser Glu Lys Cys
    130                 135                 140

His Asp Phe Gln Cys Ala Leu Leu Ala Asn Leu Phe Ala Ser Glu Gly
145                 150                 155                 160

Gln Pro Gly Lys Val Ile Glu Val Lys Ala Lys Arg Arg Thr Gly Pro
                165                 170                 175

Leu Asn Phe Val Thr Cys Met Arg Glu Thr Leu Glu Lys His Tyr Gly
            180                 185                 190

Asn Lys Pro Ile Gly Met Gly Gly Thr Phe Ile Ile Gln Lys Gly Lys
        195                 200                 205

Val Lys Ser His Ile Met Pro Ala Glu Phe Ser Ser Cys Pro Leu Asn
    210                 215                 220

Ser Asp Glu Glu Val Asn Lys Trp Leu His Phe Tyr Glu Met Lys Ala
225                 230                 235                 240

Pro Leu Val Cys Leu Pro Val Phe Val Ser Arg Asp Pro Gly Phe Asp
                245                 250                 255

Leu Arg Leu Glu His Thr His Phe Phe Ser Arg His Gly Glu Gly Gly
            260                 265                 270

His Tyr His Tyr Asp Thr Thr Pro Asp Ile Val Glu Tyr Leu Gly Tyr
        275                 280                 285

Phe Leu Pro Ala Glu Phe Leu Tyr Arg Ile Asp Gln Pro Lys Glu Thr
    290                 295                 300

His Ser Ile Gly Arg Asp
305                 310

<210>  36
<211>  251
<212>  PRT
<213>  Homo sapiens

<400>  36

Gln Asn Asp Leu Arg Pro Leu Leu Ile Glu Arg Tyr Pro Gly Ser Pro
1               5                   10                  15

Gly Ser Tyr Ala Ala Arg Gln His Ile Met Gln Arg Ile Gln Arg Leu
            20                  25                  30

Gln Ala Asp Trp Val Leu Glu Ile Asp Thr Phe Leu Ser Gln Thr Pro

35                  40                  45

Tyr Gly Tyr Arg Ser Phe Ser Asn Ile Ile Ser Thr Leu Asn Pro Thr
    50                  55                  60

Ala Lys Arg His Leu Val Leu Ala Cys His Tyr Asp Ser Lys Tyr Phe
65                  70                  75                  80

Ser His Trp Asn Asn Arg Val Phe Val Gly Ala Thr Asp Ser Ala Val
                85                  90                  95

Pro Cys Ala Met Met Leu Glu Leu Ala Arg Ala Leu Asp Lys Lys Leu
            100                 105                 110

Leu Ser Leu Lys Thr Val Ser Asp Ser Lys Pro Asp Leu Ser Leu Gln
        115                 120                 125

Leu Ile Phe Phe Asp Gly Glu Glu Ala Phe Leu His Trp Ser Pro Gln
    130                 135                 140

Asp Ser Leu Tyr Gly Ser Arg His Leu Ala Ala Lys Met Ala Ser Thr
145                 150                 155                 160

Pro His Pro Pro Gly Ala Arg Gly Thr Ser Gln Leu His Gly Met Asp
                165                 170                 175

Leu Leu Val Leu Leu Asp Leu Ile Gly Ala Pro Asn Pro Thr Phe Pro
            180                 185                 190

Asn Phe Phe Pro Asn Ser Ala Arg Trp Phe Glu Arg Leu Gln Ala Ile
        195                 200                 205

Glu His Glu Leu His Glu Leu Gly Leu Leu Lys Asp His Ser Leu Glu
    210                 215                 220

Gly Arg Tyr Phe Gln Asn Tyr Ser Tyr Gly Gly Val Ile Gln Asp Asp
225                 230                 235                 240

His Ile Pro Phe Leu Arg Arg Gly Val Pro Val
                245                 250

<210>  37
<211>  182
<212>  PRT
<213>  Homo sapiens

<400>  37

Glu Arg Asp Cys Arg Val Ser Ser Phe Arg Val Lys Glu Asn Phe Asp
1               5                   10                  15

Lys Ala Arg Phe Ser Gly Thr Trp Tyr Ala Met Ala Lys Lys Asp Pro
            20                  25                  30

Glu Gly Leu Phe Leu Gln Asp Asn Ile Val Ala Glu Phe Ser Val Asp
        35                  40                  45

Glu Thr Gly Gln Met Ser Ala Thr Ala Lys Gly Arg Val Arg Leu Leu
    50                  55                  60

Asn Asn Trp Asp Val Cys Ala Asp Met Val Gly Thr Phe Thr Asp Thr
65                  70                  75                  80

Glu Asp Pro Ala Lys Phe Lys Met Lys Tyr Trp Gly Val Ala Ser Phe
                85                  90                  95

Leu Gln Lys Gly Asn Asp Asp His Trp Ile Val Asp Thr Asp Tyr Asp
            100                 105                 110

Thr Tyr Ala Val Gln Tyr Ser Cys Arg Leu Leu Asn Leu Asp Gly Thr
        115                 120                 125

Cys Ala Asp Ser Tyr Ser Phe Val Phe Ser Arg Asp Pro Asn Gly Leu
    130                 135                 140

Pro Pro Glu Ala Gln Lys Ile Val Arg Gln Arg Gln Glu Glu Leu Cys
145                 150                 155                 160

Leu Ala Arg Gln Tyr Arg Leu Ile Val His Asn Gly Tyr Cys Asp Gly
                165                 170                 175

Arg Ser Glu Arg Asn Leu
            180

<210>  38
<211>  472
<212>  PRT
<213>  Homo sapiens

<400>  38

Met Ala Thr Lys Cys Gly Asn Cys Gly Pro Gly Tyr Ser Thr Pro Leu
1               5                   10                  15

Glu Ala Met Lys Gly Pro Arg Glu Glu Ile Val Tyr Leu Pro Cys Ile
            20                  25                  30

Tyr Arg Asn Thr Gly Thr Glu Ala Pro Asp Tyr Leu Ala Thr Val Asp
        35                  40                  45

Val Asp Pro Lys Ser Pro Gln Tyr Cys Gln Val Ile His Arg Leu Pro
    50                  55                  60

Met Pro Asn Leu Lys Asp Glu Leu His His Ser Gly Trp Asn Thr Tyr
65                  70                  75                  80

Ser Ser Cys Phe Gly Asp Ser Thr Lys Ser Arg Asn Lys Leu Val Leu

                85                  90                  95

Pro Ser Leu Ile Ser Ser Arg Ile Tyr Val Val Asp Val Gly Ser Glu
            100                 105                 110

Pro Gly Pro Gln Lys Leu His Lys Val Ile Glu Pro Lys Asp Ile His
        115                 120                 125

Ala Lys Cys Glu Leu Ala Cys Leu His Thr Ser His Cys Leu Ala Ser
    130                 135                 140

Gly Glu Val Met Ile Ser Ser Leu Gly Asp Val Lys Gly Asn Gly Lys
145                 150                 155                 160

Gly Gly Phe Val Leu Leu Asp Gly Glu Thr Phe Glu Val Lys Gly Thr
                165                 170                 175

Trp Glu Arg Pro Gly Gly Ala Ala Pro Leu Gly Tyr Asp Phe Trp Tyr
            180                 185                 190

Gln Pro Arg His Asn Val Met Ile Ser Thr Glu Trp Ala Ala Pro Asn
        195                 200                 205

Val Leu Arg Asp Gly Phe Asn Pro Ala Asp Val Glu Ala Gly Leu Tyr
    210                 215                 220

Gly Ser His Leu Tyr Val Trp Asp Trp Gln Arg His Glu Ile Val Gln
225                 230                 235                 240

Thr Leu Ser Leu Lys Asp Gly Leu Ile Pro Leu Glu Ile Arg Phe Leu
                245                 250                 255

His Asn Pro Ser Ala Thr Gln Gly Phe Val Gly Cys Ala Ser Ala Pro
            260                 265                 270

Asn Ile Gln Arg Phe Tyr Lys Thr Arg Glu Gly Thr Trp Ser Val Glu
        275                 280                 285

Lys Val Ile Gln Val Pro Pro Lys Lys Val Lys Gly Trp Leu Leu Pro
    290                 295                 300

Gly Val Pro Gly Leu Ile Thr Asp Ile Leu Leu Ser Leu Asp Asp Arg
305                 310                 315                 320

Phe Leu Tyr Phe Ser Asn Trp Leu His Gly Asp Leu Arg Gln Tyr Asp
                325                 330                 335

Ile Ser Asp Pro Gln Arg Pro Arg Leu Thr Gly Gln Leu Phe Leu Gly
            340                 345                 350

Gly Ser Ile Val Lys Gly Gly Pro Val Gln Val Leu Glu Asp Glu Glu
        355                 360                 365

Leu Lys Ser Gln Pro Glu Pro Leu Val Val Lys Gly Lys Arg Val Ala
    370                 375                 380

Gly Gly Pro Gln Met Ile Gln Leu Ser Leu Asp Gly Lys Arg Leu Tyr
385                 390                 395                 400

Ile Thr Thr Ser Leu Tyr Ser Ala Trp Glu Lys Gln Phe Tyr Pro Asp
                405                 410                 415

Leu Ile Arg Glu Gly Ser Val Met Leu Gln Val Asp Val Asp Thr Val
            420                 425                 430

Lys Gly Gly Leu Lys Leu Asn Pro Asn Cys Leu Val Asp Phe Gly Lys
        435                 440                 445

Glu Pro Leu Gly Pro Ala Leu Ala His Glu Leu Arg Tyr Pro Gly Gly
    450                 455                 460

Asp Cys Ser Ser Asp Ile Trp Ile
465                 470

<210>  39
<211>  543
<212>  PRT
<213>  Homo sapiens

<400>  39

Met Glu Gln Val Asn Glu Leu Lys Glu Lys Gly Asn Lys Ala Leu Ser
1               5                   10                  15

Val Gly Asn Ile Asp Asp Ala Leu Gln Cys Tyr Ser Glu Ala Ile Lys
            20                  25                  30

Leu Asp Pro His Asn His Val Leu Tyr Ser Asn Arg Ser Ala Ala Tyr
        35                  40                  45

Ala Lys Lys Gly Asp Tyr Gln Lys Ala Tyr Glu Asp Gly Cys Lys Thr
    50                  55                  60

Val Asp Leu Lys Pro Asp Trp Gly Lys Gly Tyr Ser Arg Lys Ala Ala
65                  70                  75                  80

Ala Leu Glu Phe Leu Asn Arg Phe Glu Glu Ala Lys Arg Thr Tyr Glu
                85                  90                  95

Glu Gly Leu Lys His Glu Ala Asn Asn Pro Gln Leu Lys Glu Gly Leu
            100                 105                 110

Gln Asn Met Glu Ala Arg Leu Ala Glu Arg Lys Phe Met Asn Pro Phe
        115                 120                 125

Asn Met Pro Asn Leu Tyr Gln Lys Leu Glu Ser Asp Pro Arg Thr Arg
    130                 135                 140

Thr Leu Leu Ser Asp Pro Thr Tyr Arg Glu Leu Ile Glu Gln Leu Arg
145                 150                 155                 160

Asn Lys Pro Ser Asp Leu Gly Thr Lys Leu Gln Asp Pro Arg Ile Met
                165                 170                 175

Thr Thr Leu Ser Val Leu Leu Gly Val Asp Leu Gly Ser Met Asp Glu
            180                 185                 190

Glu Glu Glu Ile Ala Thr Pro Pro Pro Pro Pro Pro Pro Lys Lys Glu
        195                 200                 205

Thr Lys Pro Glu Pro Met Glu Glu Asp Leu Pro Glu Asn Lys Lys Gln
    210                 215                 220

Ala Leu Lys Glu Lys Glu Leu Gly Asn Asp Ala Tyr Lys Lys Lys Asp
225                 230                 235                 240

Phe Asp Thr Ala Leu Lys His Tyr Asp Lys Ala Lys Glu Leu Asp Pro
                245                 250                 255

Thr Asn Met Thr Tyr Ile Thr Asn Gln Ala Ala Val Tyr Phe Glu Lys
            260                 265                 270

Gly Asp Tyr Asn Lys Cys Arg Glu Leu Cys Glu Lys Ala Ile Glu Val
        275                 280                 285

Gly Arg Glu Asn Arg Glu Asp Tyr Arg Gln Ile Ala Lys Ala Tyr Ala
    290                 295                 300

Arg Ile Gly Asn Ser Tyr Phe Lys Glu Glu Lys Tyr Lys Asp Ala Ile
305                 310                 315                 320

His Phe Tyr Asn Lys Ser Leu Ala Glu His Arg Thr Pro Asp Val Leu
                325                 330                 335

Lys Lys Cys Gln Gln Ala Glu Lys Ile Leu Lys Glu Gln Glu Arg Leu
            340                 345                 350

Ala Tyr Ile Asn Pro Asp Leu Ala Leu Glu Glu Lys Asn Lys Gly Asn
        355                 360                 365

Glu Cys Phe Gln Lys Gly Asp Tyr Pro Gln Ala Met Lys His Tyr Thr
    370                 375                 380

Glu Ala Ile Lys Arg Asn Pro Lys Asp Ala Lys Leu Tyr Ser Asn Arg
385                 390                 395                 400

Ala Ala Cys Tyr Thr Lys Leu Leu Glu Phe Gln Leu Ala Leu Lys Asp

405                 410                 415

Cys Glu Glu Cys Ile Gln Leu Glu Pro Thr Phe Ile Lys Gly Tyr Thr
            420                 425                 430

Arg Lys Ala Ala Ala Leu Glu Ala Met Lys Asp Tyr Thr Lys Ala Met
        435                 440                 445

Asp Val Tyr Gln Lys Ala Leu Asp Leu Asp Ser Ser Cys Lys Glu Ala
    450                 455                 460

Ala Asp Gly Tyr Gln Arg Cys Met Met Ala Gln Tyr Asn Arg His Asp
465                 470                 475                 480

Ser Pro Glu Asp Val Lys Arg Arg Ala Met Ala Asp Pro Glu Val Gln
                485                 490                 495

Gln Ile Met Ser Asp Pro Ala Met Arg Leu Ile Leu Glu Gln Met Gln
            500                 505                 510

Lys Asp Pro Gln Ala Leu Ser Glu His Leu Lys Asn Pro Val Ile Ala
        515                 520                 525

Gln Lys Ile Gln Lys Leu Met Asp Val Gly Leu Ile Ala Ile Arg
    530                 535                 540

<210>  40
<211>  318
<212>  PRT
<213>  Homo sapiens

<400>  40

Met Ser Ser Ser Pro Val Lys Arg Gln Arg Met Glu Ser Ala Leu Asp
1               5                   10                  15

Gln Leu Lys Gln Phe Thr Thr Val Val Ala Asp Thr Gly Asp Phe His
            20                  25                  30

Ala Ile Asp Glu Tyr Lys Pro Gln Asp Ala Thr Thr Asn Pro Ser Leu
        35                  40                  45

Ile Leu Ala Ala Ala Gln Met Pro Ala Tyr Gln Glu Leu Val Glu Glu
    50                  55                  60

Ala Ile Ala Tyr Gly Arg Lys Leu Gly Gly Ser Gln Glu Asp Gln Ile
65                  70                  75                  80

Lys Asn Ala Ile Asp Lys Leu Phe Val Leu Phe Gly Ala Glu Ile Leu
                85                  90                  95

Lys Lys Ile Pro Gly Arg Val Ser Thr Glu Val Asp Ala Arg Leu Ser
            100                 105                 110

Phe Asp Lys Asp Ala Met Val Ala Arg Ala Arg Arg Leu Ile Glu Leu
        115                 120                 125

Tyr Lys Glu Ala Gly Ile Ser Lys Asp Arg Ile Leu Ile Lys Leu Ser
    130                 135                 140

Ser Thr Trp Glu Gly Ile Gln Ala Gly Lys Glu Leu Glu Glu Gln His
145                 150                 155                 160

Gly Ile His Cys Asn Met Thr Leu Leu Phe Ser Phe Ala Gln Ala Val
                165                 170                 175

Ala Cys Ala Glu Ala Gly Val Thr Leu Ile Ser Pro Phe Val Gly Arg
            180                 185                 190

Ile Leu Asp Trp His Val Ala Asn Thr Asp Lys Lys Ser Tyr Glu Pro
        195                 200                 205

Leu Glu Asp Pro Gly Val Lys Ser Val Thr Lys Ile Tyr Asn Tyr Tyr
    210                 215                 220

Lys Lys Phe Ser Tyr Lys Thr Ile Val Met Gly Ala Ser Phe Arg Asn
225                 230                 235                 240

Thr Gly Glu Ile Lys Ala Leu Ala Gly Cys Asp Phe Leu Thr Ile Ser
                245                 250                 255

Pro Lys Leu Leu Gly Glu Leu Leu Gln Asp Asn Ala Lys Leu Val Pro
            260                 265                 270

Val Leu Ser Ala Lys Pro Lys Pro Val Thr Trp Lys Lys Ser Thr Trp
        275                 280                 285

Met Arg Ser Leu Ser Val Gly Cys Thr Thr Arg Thr Arg Trp Leu Trp
    290                 295                 300

Arg Ser Ser Leu Thr Gly Ser Ala Ser Leu Pro Leu Met Gln
305                 310                 315

<210>  41
<211>  534
<212>  PRT
<213>  Homo sapiens

<400>  41

Met Pro Tyr Glu Ile Lys Lys Val Phe Ala Ser Leu Pro Gln Val Glu
1               5                   10                  15

Arg Gly Val Ser Lys Ile Ile Gly Gly Asp Pro Lys Gly Asn Asn Phe
            20                  25                  30

Leu Tyr Thr Asn Gly Lys Cys Val Ile Leu Arg Asn Ile Asp Asn Pro

        35                  40                  45

Ala Leu Ala Asp Ile Tyr Thr Glu His Ala His Gln Val Val Val Ala
    50                  55                  60

Lys Tyr Ala Pro Ser Gly Phe Tyr Ile Ala Ser Gly Asp Val Ser Gly
65                  70                  75                  80

Lys Leu Arg Ile Trp Asp Thr Thr Gln Lys Glu His Leu Leu Lys Tyr
                85                  90                  95

Glu Tyr Gln Pro Phe Ala Gly Lys Ile Lys Asp Ile Ala Trp Thr Glu
            100                 105                 110

Asp Ser Lys Arg Ile Ala Val Val Gly Glu Gly Arg Glu Lys Phe Gly
        115                 120                 125

Ala Val Phe Leu Trp Asp Ser Gly Ser Ser Val Gly Glu Ile Thr Gly
    130                 135                 140

His Asn Lys Val Ile Asn Ser Val Asp Ile Lys Gln Ser Arg Pro Tyr
145                 150                 155                 160

Arg Leu Ala Thr Gly Ser Asp Asp Asn Cys Ala Ala Phe Phe Glu Gly
                165                 170                 175

Pro Pro Phe Lys Phe Lys Phe Thr Ile Gly Asp His Ser Arg Phe Val
            180                 185                 190

Asn Cys Val Arg Phe Ser Pro Asp Gly Asn Arg Phe Ala Thr Ala Ser
        195                 200                 205

Ala Asp Gly Gln Ile Tyr Ile Tyr Asp Gly Lys Thr Gly Glu Lys Val
    210                 215                 220

Cys Ala Leu Gly Gly Ser Lys Ala His Asp Gly Gly Ile Tyr Ala Ile
225                 230                 235                 240

Ser Trp Ser Pro Asp Ser Thr His Leu Leu Ser Ala Ser Gly Asp Lys
                245                 250                 255

Thr Ser Lys Ile Trp Asp Val Ser Val Asn Ser Val Val Ser Thr Phe
            260                 265                 270

Pro Met Gly Ser Thr Val Leu Asp Gln Gln Leu Gly Cys Leu Trp Gln
        275                 280                 285

Lys Asp His Leu Leu Ser Val Ser Leu Ser Gly Tyr Ile Asn Tyr Leu
    290                 295                 300

Asp Arg Asn Asn Pro Ser Lys Pro Leu His Val Ile Lys Gly His Ser
305                 310                 315                 320

Lys Ser Ile Gln Cys Leu Thr Val His Lys Asn Gly Gly Lys Ser Tyr
                325                 330                 335

Ile Tyr Ser Gly Ser His Asp Gly His Ile Asn Tyr Trp Asp Ser Glu
            340                 345                 350

Thr Gly Glu Asn Asp Ser Phe Ala Gly Lys Gly His Thr Asn Gln Val
        355                 360                 365

Ser Arg Met Thr Val Asp Glu Ser Gly Gln Leu Ile Ser Cys Ser Met
    370                 375                 380

Asp Asp Thr Val Arg Tyr Thr Ser Leu Met Leu Arg Asp Tyr Ser Gly
385                 390                 395                 400

Gln Gly Val Val Lys Leu Asp Val Gln Pro Lys Cys Val Ala Val Gly
                405                 410                 415

Pro Gly Gly Tyr Ala Val Val Val Cys Ile Gly Gln Ile Val Leu Leu
            420                 425                 430

Lys Asp Gln Arg Lys Cys Phe Ser Ile Asp Asn Pro Gly Tyr Glu Pro
        435                 440                 445

Glu Val Val Ala Val His Pro Gly Gly Asp Thr Val Ala Ile Gly Gly
    450                 455                 460

Val Asp Gly Asn Val Arg Leu Tyr Ser Ile Leu Gly Thr Thr Leu Lys
465                 470                 475                 480

Asp Glu Gly Lys Leu Leu Glu Ala Lys Gly Pro Val Thr Asp Val Ala
                485                 490                 495

Tyr Ser His Asp Gly Ala Phe Leu Ala Val Cys Asp Ala Ser Lys Val
            500                 505                 510

Val Thr Val Phe Ser Val Ala Asp Gly Tyr Ser Glu Asn Asn Val Phe
        515                 520                 525

Tyr Gly His His Glu Lys
    530